(19) Europäisches Patentamt
European Patent Office
Office européen des brevets

(11) Veröffentlichungsnummer: **0 045 521**
**B1**

# (12) EUROPÄISCHE PATENTSCHRIFT

(45) Veröffentlichungstag der Patentschrift: **27.06.84**

(21) Anmeldenummer: **81106139.9**

(22) Anmeldetag: **05.08.81**

(51) Int. Cl.³: **C 07 D 487/04**, A 61 K 31/55 // C07D231/12, C07D231/14, (C07D487/04, 231/00, 223/00)

## (54) Pyrazolobenzazepine, Verfahren zu ihrer Herstellung und diese enthaltende Arzneimittel.

(30) Priorität: **05.08.80 US 175552**
**23.07.81 US 286122**

(43) Veröffentlichungstag der Anmeldung: **10.02.82 Patentblatt 82/6**

(45) Bekanntmachung des Hinweises auf die Patenterteilung: **27.06.84 Patentblatt 84/26**

(84) Benannte Vertragsstaaten: **BE CH DE FR GB IT LI NL SE**

(56) Entgegenhaltungen: **DE - A - 2 524 048**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Field, George Francis, 33 McKinley Avenue, West Caldwell, New Jersey (US)**
Erfinder: **Fryer, Rodney Ian, 5 Eton Drive, North Caldwell, New Jersey (US)**
Erfinder: **Trybulski, Eugene J., 13 Homer Street, Parsippany, New Jersey (US)**
Erfinder: **Walser, Armin, 19 Crane Avenue, West Caldwell, New Jersey (US)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 045 521 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft Pyrazolobenzazepine der allgemeinen Formeln

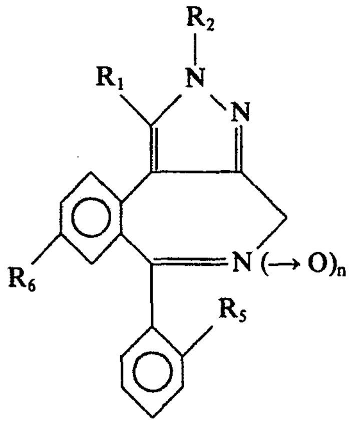

(I)

und

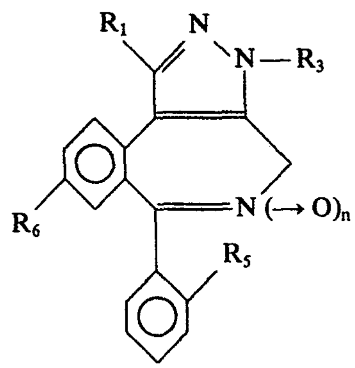

(II)

worin $R_1$ Wasserstoff oder niederes Alkyl, $R_2$ und $R_3$ Wasserstoff, niederes Alkyl, Hydroxy-$C_2$- bis $C_7$-Alkyl, $C_2$- bis $C_7$-Carbonsäureester und -amide oder die Gruppe $-COR_{11}$, $R_{11}$ Alkoxy, Amino oder mono-niederes Alkylamino, $R_6$ Nitro oder Halo mit einer Ordnungszahl, welche nicht größer als 35 ist, $R_5$ Wasserstoff oder Halo mit einer Ordnungszahl, welche nicht größer als 35 ist, und n die Zahl 0 oder 1 bedeuten,
und pharmazeutisch annehmbare Salze davon.

In DE-A1-2 524 048 sind 6-Aryl-1,4-dihydro- oder -2,4-dihydro-pyrazolo[4,3-d][2]benzazepine beschrieben, welche antidepressive Effekte vom Imipramin-Typus und insbesondere anxiolytische Wirkungen von langer Dauer zeigen.

Die in der vorliegenden Beschreibung verwendeten Ausdrücke »Halo« oder »Halogen« oder »Halogenid« beziehen sich, sofern nichts anderes angegeben ist, auf Chlor, Brom und Fluor.

Der Ausdruck »niederes Alkyl« bezeichnet geradkettige oder verzweigte $C_1$- bis $C_7$-Kohlenwasserstoffreste, wie Methyl, Äthyl, Propyl, Isopropyl, Butyl etc.

Der Ausdruck »$C_2$- bis $C_7$-Carbonsäureester und Amide davon« bezeichnet Substituenten der Formel $C_1$- bis $C_6$-Alkyl-$COR_{15}$, worin $R_{15}$ Alkoxy, Amino oder substituiertes Amino bedeutet. Substituiertes Amino bezeichnet eine $NH_2$-Gruppe, welche durch niederes Alkyl mono- oder disubstituiert sein kann, z. B. Methylamino- oder Dimethylaminogruppen.

Erfindungsgemäß können die Verbindungen der Formeln I und II und ihre pharmazeutisch annehmbaren Säureadditionssalze dadurch hergestellt werden, daß man

a) eine Verbindung der allgemeinen Formel

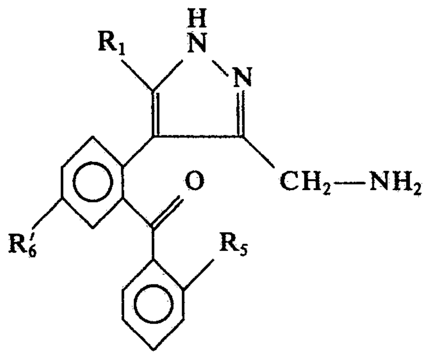

(A)

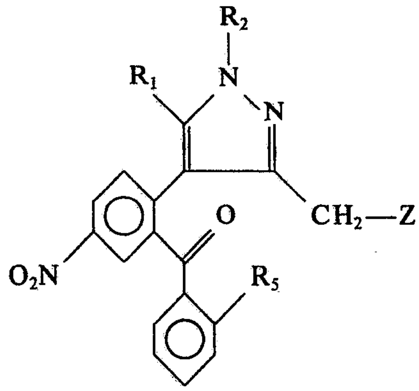

(B)

oder

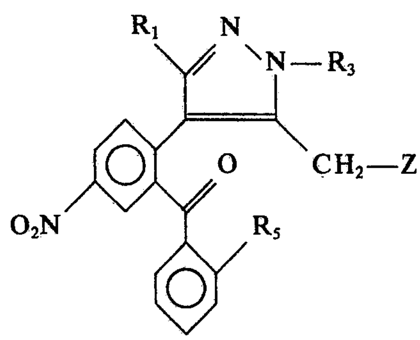

(C)

worin $R_1$, $R_2$, $R_3$ und $R_5$ obige Bedeutung besitzen, $R_6'$ Halo und Z $-NH_2$ oder $-NHOH$ bedeuten, cyclisiert, oder

b) eine Verbindung der Formel I, worin $R_2$ Wasserstoff bedeutet, zuerst mit einer Base und anschließend mit einem einen niederen Alkylrest liefernden Mittel oder einem Halo-$C_2$- bis $C_7$-Carbonsäureester behandelt, oder

c) eine Verbindung der Formel I oder II, worin $R_2$ bzw. $R_3$ $C_1$- bis $C_6$-Alkyl-COO-niederes Alkyl bedeutet, mit Ammoniak oder einem mono- oder di-niederen Alkylamin behandelt, oder

d) eine Verbindung der Formel I oder II, worin $R_2$ bzw. $R_3$ $C_1$- bis $C_6$-Alkyl-COO-niederes Alkyl und $R_6$ Halo bedeuten, mit einem Metallhydrid-Reduktionsmittel behandelt, oder

e) eine Verbindung der Formel I, worin $R_2$ Wasserstoff und $R_6$ Halo bedeuten, mit einem niederen Alkyl- oder Alkalimetallisocyanat oder zuerst mit einer Base und anschließend mit einem niederen Alkyl-Haloformat behandelt, oder

f) eine Verbindung der Formel I oder II, worin n die Zahl 0 bedeutet, zu einer Verbindung der Formel I oder II, worin n die Zahl 1 bedeutet, oxydiert, oder

g) eine Verbindung der Formel I oder II in ein pharmazeutisch annehmbares Säureadditionssalz überführt.

Die obigen Verfahrensvarianten und die Herstellung der darin benötigten Ausgangsstoffe werden durch die nachfolgenden Reaktionsschemata illustriert:

Schema I

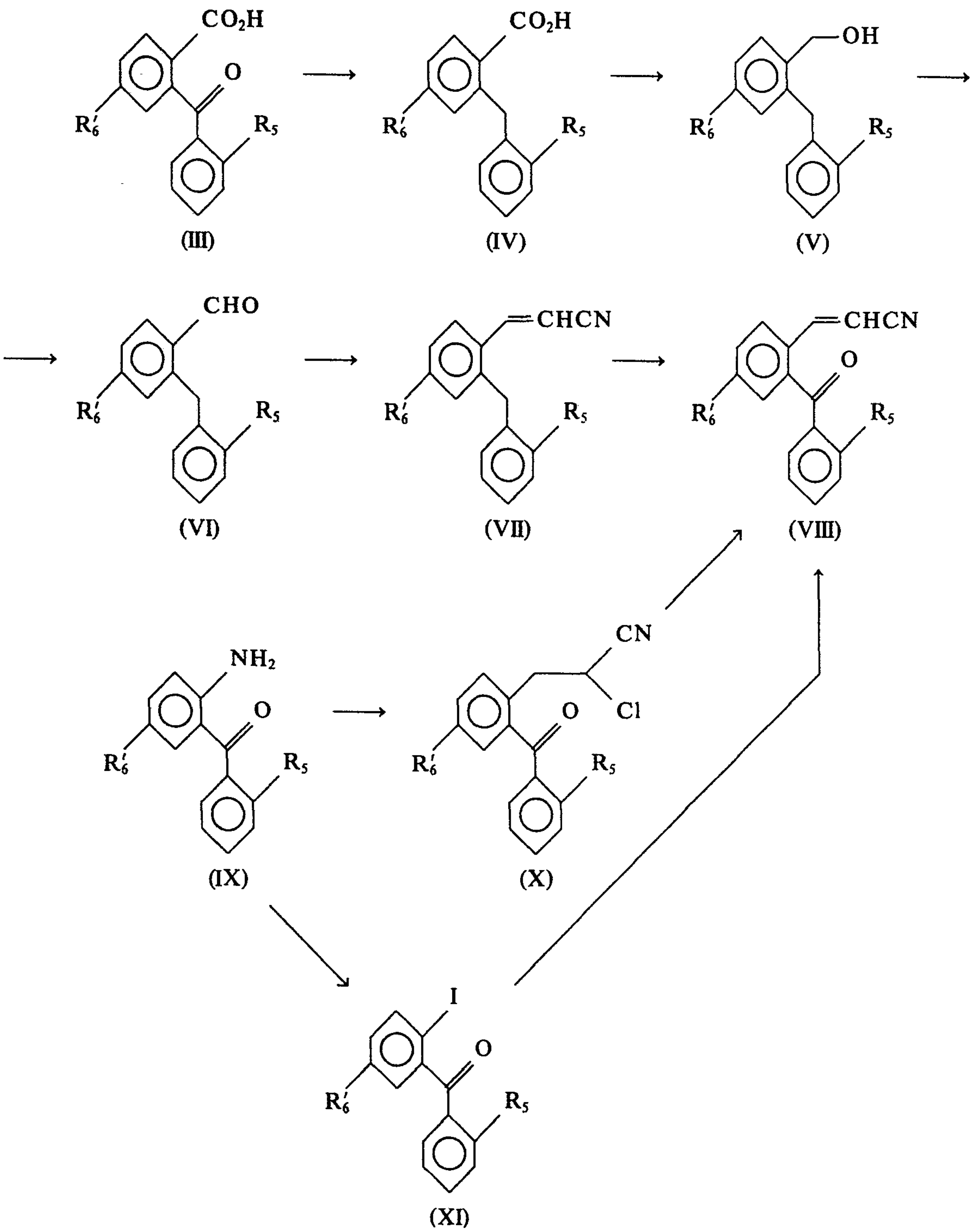

(III) (IV) (V) (VI) (VII) (VIII) (IX) (X) (XI)

worin $R_5$ obige Bedeutung besitzt und $R_6'$ Halo bedeutet.

III → IV

Eine Verbindung der Formel III, ein bekanntes Ausgangsmaterial, kann in konzentriertem Ammoniak mit Zinkstaub und Kupfersulfat umgesetzt werden. Die Reaktionstemperatur variiert in einem Bereich von Raumtemperatur bis 100° C und beträgt vorzugsweise etwa 100° C.

IV → V

Eine Verbindung der Formel IV kann mit einem Metallhydrid, wie Lithiumaluminiumhydrid, oder einem Boran in einem ätherischen Lösungsmittel, wie Diäthyläther oder Tetrahydrofuran, umgesetzt werden. Die Reaktionstemperatur liegt in einem Bereich von −78° C bis Raumtemperatur und beträgt vorzugsweise 0° C.

V → VI

Eine Verbindung der Formel V kann unter Verwendung von Methylenchlorid als Lösungsmittel mit Pyridiniumchlorchromat, Mangandioxid oder einem anderen geeigneten Oxidationsmittel umgesetzt werden. Die Reaktionstemperatur liegt in einem Bereich von 0° C bis Siedetemperatur des gewählten Lösungsmittels und liegt vorzugsweise bei etwa Raumtemperatur.

VI → VII

Eine Verbindung der Formel VI kann in Gegenwart einer starken Base, wie Natriumhydrid, Natriumamid etc., und unter Verwendung eines ätherischen Lösungsmittels, wie Tetrahydrofuran, mit Diäthylcyanomethylphosphonat umgesetzt werden. Die Reaktionstemperatur variiert in einem Bereich von 0° C bis Raumtemperatur und liegt vorzugsweise bei etwa Raumtemperatur.

VII → VIII

Eine Verbindung der Formel VII kann in einer Mischung von Essigsäure und Methylenchlorid mit Chromtrioxid oder einem davon abgeleiteten Oxidationsmittel umgesetzt werden. Die Reaktionstemperatur variiert in einem Bereich von 0° C bis 60° C und liegt vorzugsweise bei Raumtemperatur.

IX → X

Die Verbindungen der Formel IX, d. h. die Aminoketone, sind bekannte Ausgangsstoffe. Eine solche Verbindung kann in Gegenwart von Acetonitril, einem niederen Alkylnitrit und Kupferchlorid mit Acrylnitril umgesetzt werden. Die Reaktionstemperatur kann in einem Bereich von 0°C bis 40°C variieren und liegt vorzugsweise bei etwa Raumtemperatur.

X → VIII

Eine Verbindung der Formel X kann mit einer Mischung aus einem Alkalimetall-, z. B. Lithium-, Natrium- oder Kalium-, -carbonat und einem -bicarbonat, vorzugsweise mit einer Mischung von einem Teil Kaliumcarbonat und drei Teilen Kaliumbicarbonat, umgesetzt werden. Unter den geeigneten Lösungsmitteln sind Dimethylsulfoxid, Dimethylformamid oder $C_1$- bis $C_4$-Alkohole, z. B. Methanol. Die Reaktionstemperatur variiert in einem Bereich von 20° C bis 50° C und liegt vorzugsweise bei Raumtemperatur. Bei der obigen Reaktion handelt es sich um eine dem Fachmann bekannte Dehydrohalogenierung.

IX → XI

Eine Verbindung der Formel IX kann unter Verwendung von Natriumnitrit in Schwefelsäure diazotiert werden; das erhaltene Diazoniumsalz kann als entsprechendes Tetrafluoroborat ausgewählt werden. Man kann das erhaltene Salz in Wasser aufschlämmen und mit wäßrigem Kaliumiodid bei Raumtemperatur behandeln, wobei man ein Jodbenzophenon der Formel XI erhält.

XI → VIII

Eine Verbindung der Formel XI kann in Gegenwart eines Palladium(II)salzes, wie dem Acetat, unter Verwendung von Acetonitril oder einem aromatischen Kohlenwasserstoff, wie Toluol, als Lösungsmittel mit Acrylnitril umgesetzt werden. Die Reaktionstemperatur variiert in einem Bereich von 60° C bis Siedetemperatur des Lösungsmittels und liegt vorzugsweise bei der Siedetemperatur.

Schema II

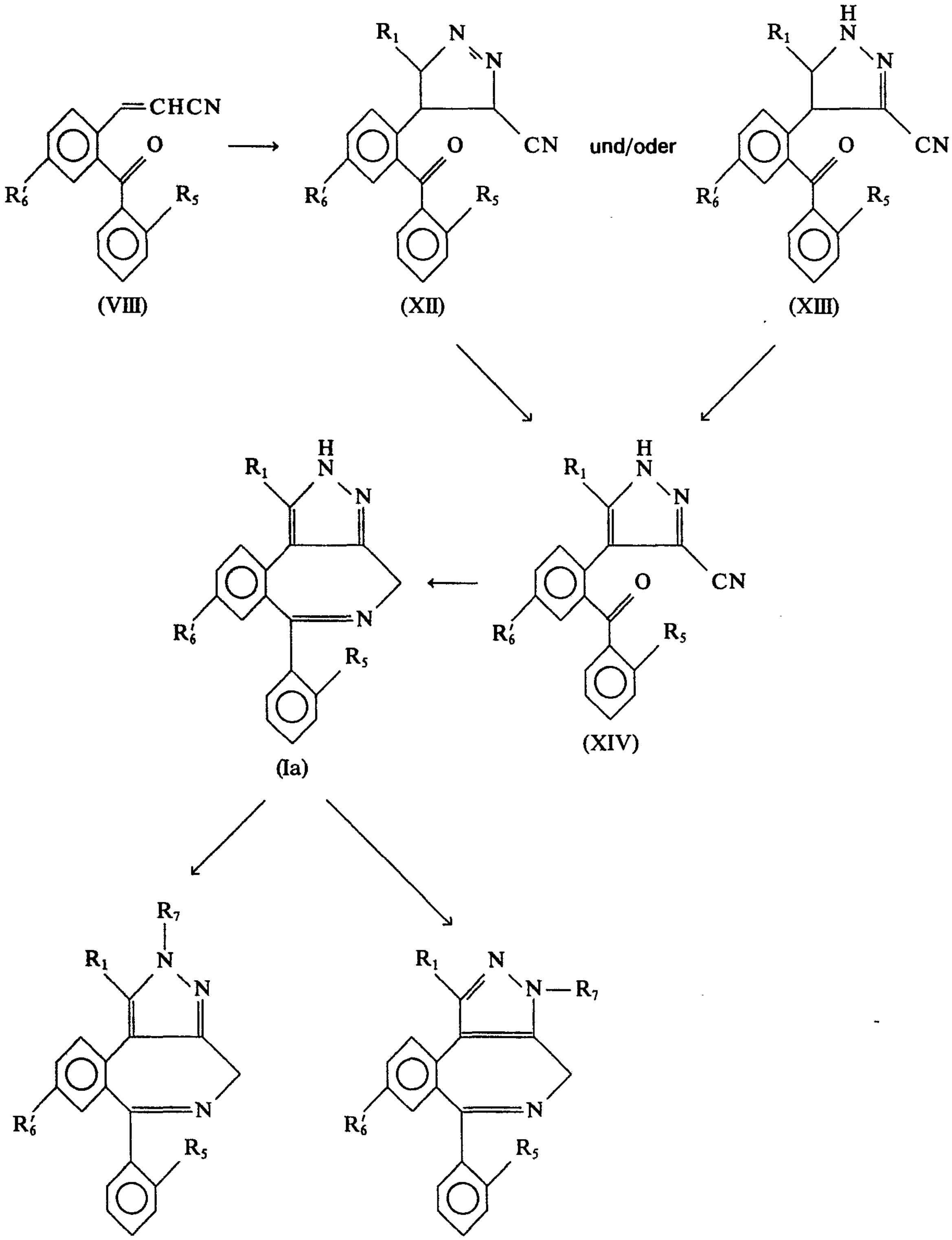

(VIII) (XII) und/oder (XIII)

(XIV)

(Ia)

(Ib) (IIb)

worin $R_1$ und $R_5$ obige Bedeutung besitzen, $R_6'$ Halogen und $R_7$ niederes Alkyl oder $C_2$- bis $C_7$-Carbonsäureester bedeuten.

VIII → XII und/oder XIII

Eine Verbindung der Formel VIII kann mit einem Diazoalkan der Formel

$R_1-CHN_2$,

worin $R_1$ obige Bedeutung besitzt,
umgesetzt werden.

Unter den geeigneten Lösungsmitteln sind ätherische Lösungsmittel, wie Tetrahydrofuran oder Dioxan, wobei man Methylenchlorid als Lösungsvermittler verwenden kann. Die Reaktion wird zwischen 0°C und Raumtemperatur, vorzugsweise bei etwa Raumtemperatur, durchgeführt.

XII und/oder XIII → XIV

Eine Verbindung der Formel XII und/oder XIII kann anschließend in einem verträglichen Lösungsmittel, wie Toluol, Tetrahydrofuran oder Methylenchlorid, mit einem Dehydrierungsmittel, wie Mangandioxid, umgesetzt werden. Die Reaktionstemperatur kann in einem Bereich von Raumtemperatur bis Siedetemperatur des gewählten Lösungsmittels variieren und liegt vorzugsweise bei der Siedetemperatur.

Andererseits kann man eine Verbindung der Formel XII und/oder XIII in einem halogenierten Kohlenwasserstoff, wie Methylenchlorid oder Chloroform, als Lösungsmittel und bei etwa Raumtemperatur mit Brom umsetzen, und die anschließende Dehydrohalogenierung durch Erhitzen oder durch Behandeln mit einer milden Base bewerkstelligen. Zu diesem Zweck wird die Reaktionsmischung entweder auf die Siedetemperatur des Lösungsmittels erhitzt oder mit einer milden Base, wie einem Alkalimetallbicarbonat oder einem Trialkylamin, behandelt.

XIV → Ia

Eine Verbindung der Formel XIV kann in Gegenwart eines Übergangsmetallkatalysators, wie Raney-Nickel, und unter Verwendung von Eisessig als Lösungsmittel bei Drucken von Atmosphärendurck bis 5 Atmosphären mit Wasserstoff umgesetzt werden. Die Reaktionstemperatur liegt bei etwa Raumtemperatur. Die Reduktion einer Verbindung der Formel XIV führt in einem ersten Schritt zu einem Amin der Formel A, das anschließend zu einer Verbindung der Formel Ia cyclisiert.

Ia → Ib und IIb

Eine Verbindung der Formel Ia kann in einem Lösungsmittel, wie Tetrahydrofuran, Dioxan, Dimethylformamid oder Dimethylsulfoxid zuerst mit einem Alkalimetallalkoxid, wie Natrium- oder Kaliummethoxid oder -äthoxid, oder einem Lithium-di-niederen Alkylamid und anschließend mit einem Alkylierungsmittel, wie einem niederen Alkylhalogenid oder einem -sulfonat umgesetzt werden. Die zweite Reaktion kann auch mit einem Haloester, wie Äthylbromacetat oder Äthyl-3-brompropionat durchgeführt werden, wobei man eine Verbindung erhält, worin $R_7$ $C_2$- bis $C_7$-Carbonsäureester bedeutet. Die Reaktion kann in einem Temperaturbereich von 0°C bis Raumtemperatur durchgeführt werden und liegt vorzugsweise bei etwa 0°C.

Schema III

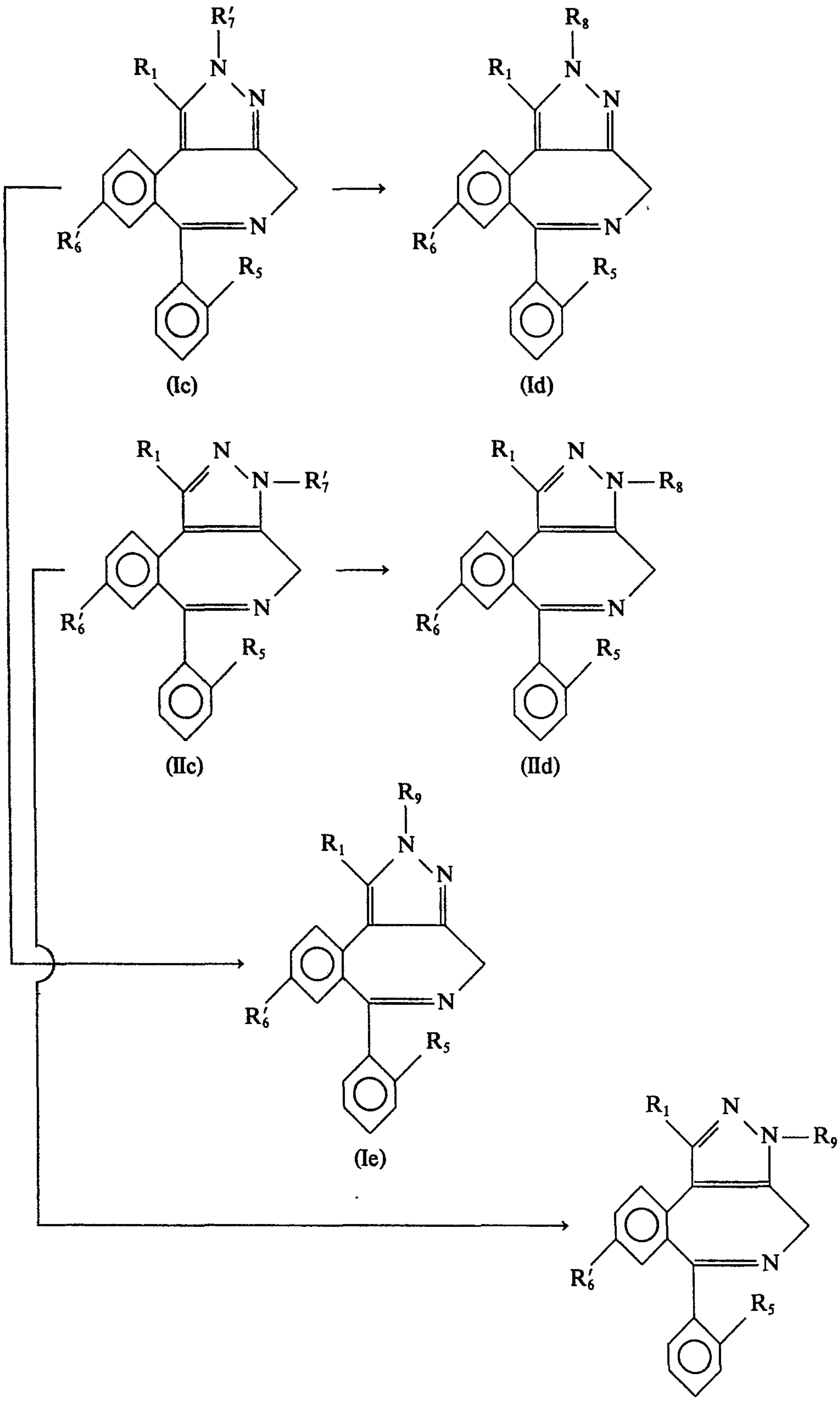

(Ic) (Id)

(IIc) (IId)

(Ie)

(IIe)

worin $R_1$ und $R_5$ obige Bedeutung besitzen, $R_6'$ Halogen, $R_7'$ $C_2$- bis $C_7$-Carbonsäureester, $R_8$ Carbonsäureamid und $R_9$ Hydroxy-$C_2$- bis $C_7$-Alkyl bedeuten.

Ic → Id und IIc → IId

Die Verbindungen der Formeln Ic und IIc können in einem $C_1$- bis $C_4$-Alkohol als Lösungsmittel mit Ammoniak oder einem mono- oder di-niederen Alkylamin und einer katalytischen Menge des entsprechenden Hydrochlorides umgesetzt werden. Die Reaktion wird bei etwa 100° C durchgeführt, wobei man in einer Druckapparatur arbeitet, um die flüchtigen Reaktanden zurückzuhalten.

Ic → Ie und IIc → IIe

Die Verbindungen der Formeln Ic und IIc können in einem ätherischen Lösungsmittel, wie Tetrahydrofuran oder Dioxan, mit einem Metallhydrid-Reduktonsmittel, wie Lithiumaluminiumhydrid umgesetzt werden. Die Reaktion wird in einem Temperaturbereich von −78° C bis Raumtemperatur durchgeführt, vorzugsweise bei etwa 0° C.

Schema IV

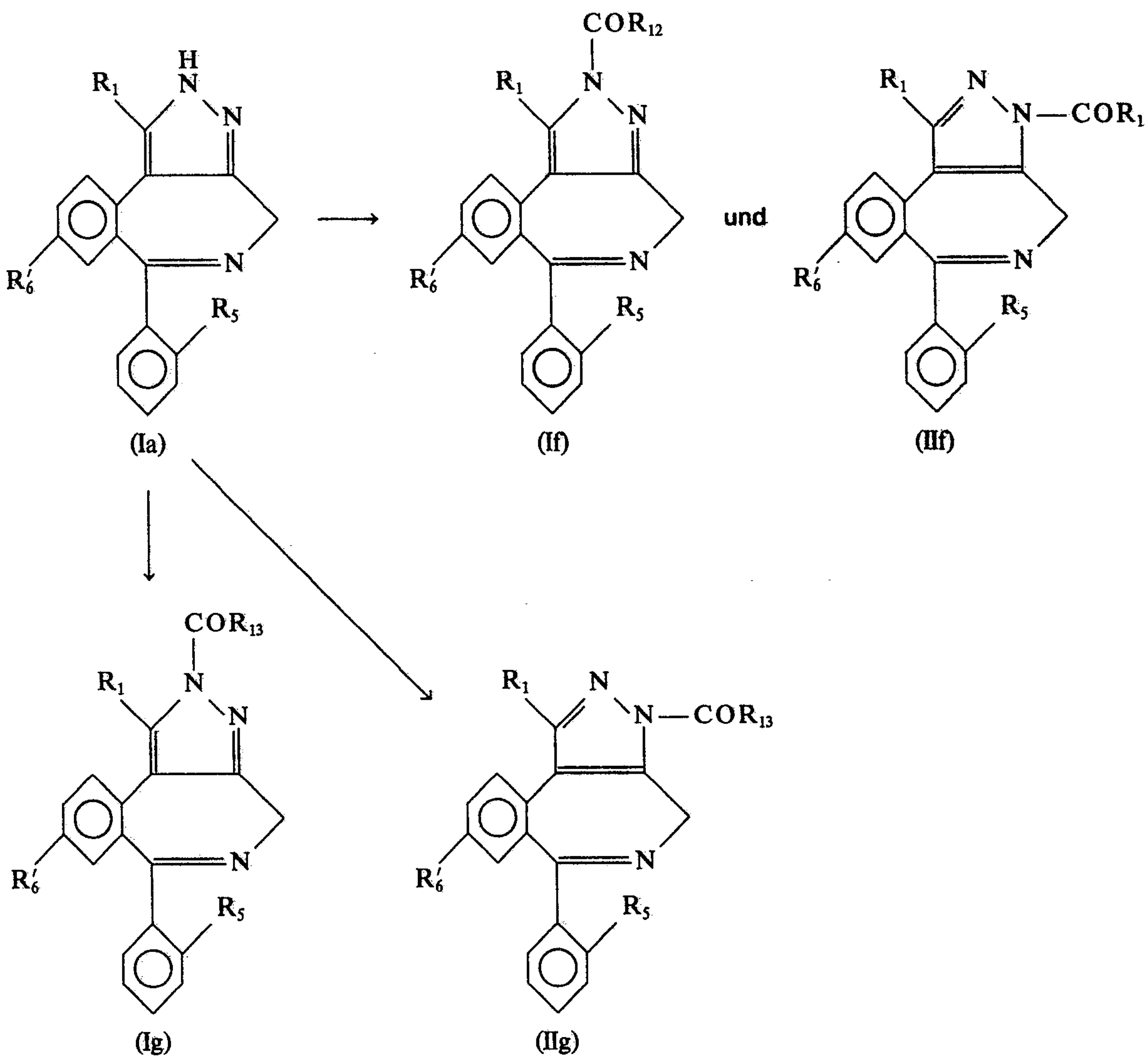

(Ia) (If) und (IIf)

(Ig) (IIg)

worin $R_1$ und $R_5$ obige Bedeutung besitzen, $R_6'$ Halogen, $R_{12}$ Amino oder mono-niederes Alkylamino und $R_{13}$ niederes Alkoxy bedeuten.

Ia → If und IIf

Eine Verbindung der Formel Ia kann in einem ätherischen Lösungsmittel, wie Tetrahydrofuran oder Dioxan, oder in einem chlorierten Kohlenwasserstoff, wie Methylenchlorid oder Chloroform, mit

einem niederen Alkyl- oder Alkalimetallisocyanat umgesetzt werden. Die Reaktionstemperatur liegt in einem Bereich von −20° C bis Raumtemperatur und beträgt vorzugsweise etwa 0° C.

Ia → Ig und IIg

Eine Verbindung der Formel Ia kann in einem ätherischen Lösungsmittel, wie Tetrahydrofuran oder Dioxan, oder in Dimethylformamid zuerst mit einem Alkalimetallalkoxid, wie Natrium- oder Kaliumäthoxid oder -butoxid, oder mit einem Lithium-di-niederen Alkylamid und anschließend mit einem Haloformat, wie Methylchlorformat, Benzylchlorformat etc., umgesetzt werden. Die Reaktionstemperatur liegt zwischen −78° C und Raumtemperatur und beträgt vorzugsweise etwa 0° C.

Schema V

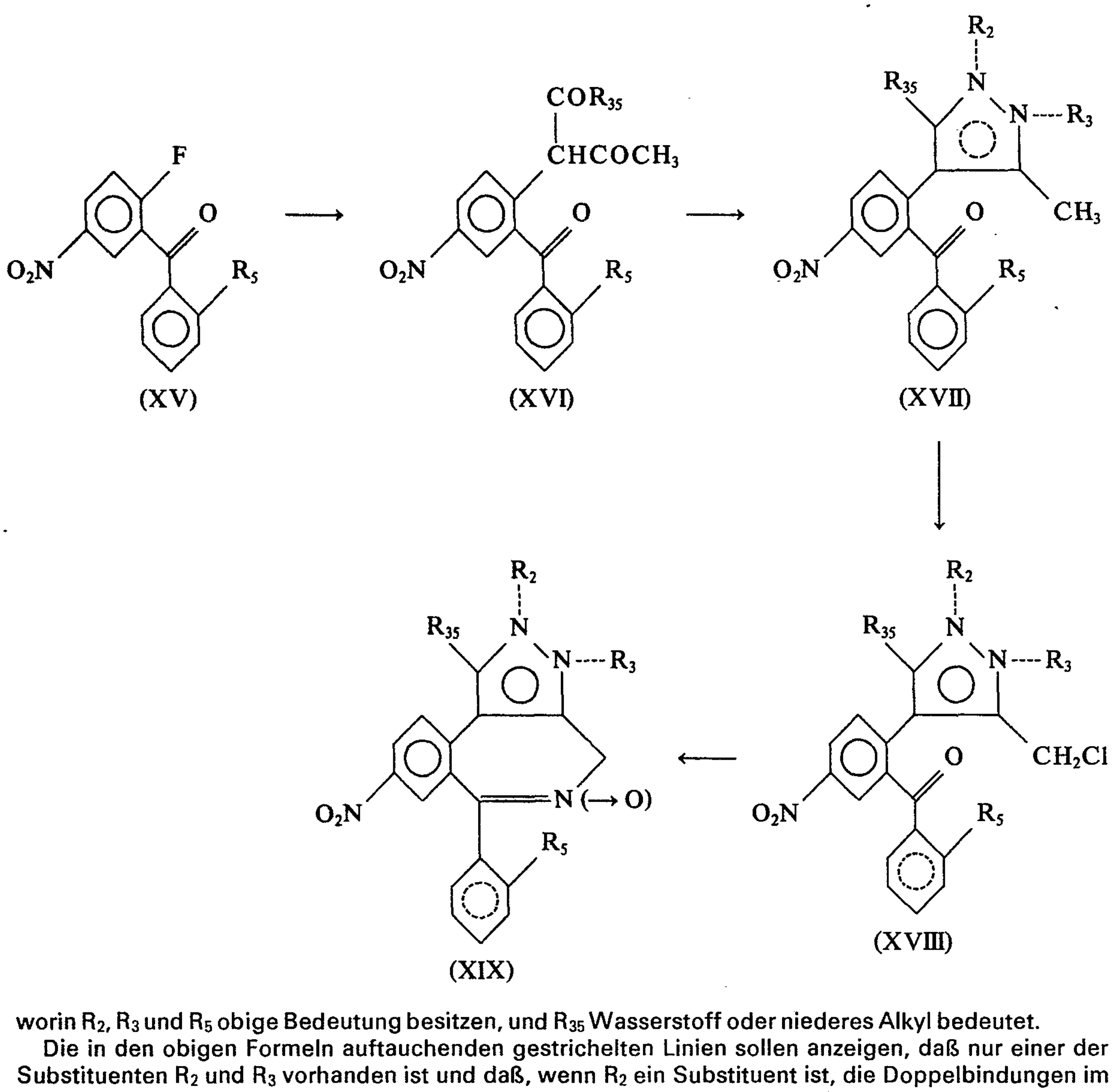

(XV) (XVI) (XVII) (XVIII) (XIX)

worin $R_2$, $R_3$ und $R_5$ obige Bedeutung besitzen, und $R_{35}$ Wasserstoff oder niederes Alkyl bedeutet.

Die in den obigen Formeln auftauchenden gestrichelten Linien sollen anzeigen, daß nur einer der Substituenten $R_2$ und $R_3$ vorhanden ist und daß, wenn $R_2$ ein Substituent ist, die Doppelbindungen im Pyrazolring sich in der 1,2- und 4,5-Stellung befinden, und daß, wenn $R_3$ ein Substituent ist, die Doppelbindungen im Pyrazolring sich in der 2,3- und 1,5-Stellung befinden.

In Analogie zu den Reaktionsschritten Ia → Ib, Ia → Iib, Ic → Id und IIc → IId können Verbindungen der Formel XIX, worin $R_2$ und $R_3$ Wasserstoff bedeuten, so modifiziert werden, daß $R_2$ und $R_3$ niederes Alkyl, oder $C_2$- bis $C_7$-Carbonsäureester oder -amide bedeuten.

XV → XVI

Die Verbindungen der Formel XV können nach an sich bekannten Methoden hergestellt werden.

Eine Verbindung der Formel XV kann in Gegenwart einer starken Base, wie Natriumhydrid oder Kalium- oder Natriummethoxid, mit einer Dicarbonylverbindung der Formel

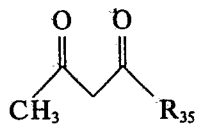

worin $R_{35}$ obige Bedeutung besitzt, wie Acetylaceton, Propionylaceton oder Acetylacetaldehyd, umgesetzt werden. Geeignete Lösungsmittel sind polare aprotische Lösungsmittel, wie Dimethylsulfoxid, Dimethylformamid oder Tetrahydrofuran. Die Reaktion kann in einem Temperaturbereich von −10° C bis 100° C durchgeführt werden, wird jedoch vorzugsweise bei etwa Raumtemperatur durchgeführt.

XVI → XVII

Eine Verbindung der Formel XVI kann anschließend mit einem Hydrazinsalz oder einem substituierten Hydrazinsalz, d. h. mit einem Salz von Hydrazin, das mit dem gewünschten Rest $R_2$ oder $R_3$ substituiert ist, umgesetzt werden. Als Salze kommen dabei Mineralsäuresalze, wie die Hydrochloride oder Sulfate in Frage. Unter den geeigneten Lösungsmitteln sind wäßrige $C_1$- bis $C_4$-Alkohole. Die Reaktion kann zwischen Raumtemperatur und Siedetemperatur durchgeführt werden. Die Reaktionstemperatur liegt vorzugsweise bei der Siedetemperatur des gewählten Lösungsmittels.

XVII → XVIII

Eine Verbindung der Formel XVII kann anschließend mit einem Halogenierungsmittel, wie t-Butylhypochlorit oder N-Bromsuccinimid umgesetzt werden. Unter den geeigneten Lösungsmitteln sind nichtpolare Lösungsmittel, wie Methylenchlorid, Benzol oder Toluol. Die Reaktion kann zwischen 0° C und Siedetemperatur des gewählten Lösungsmittels durchgeführt werden, wobei man vorzugsweise bei etwa Raumtemperatur arbeitet.

XVIII → XIX

Eine Verbindung der Formel XVIII kann in Gegenwart eines Lösungsmittels, wie Tetrahydrofuran, Dimethylformamid, Dimethylsulfoxid oder Methylenchlorid, mit Ammoniak oder Hydroxylamin umgesetzt werden. Die Reaktion kann in einem Bereich von −40° C bis Siedetemperatur durchgeführt werden, vorzugsweise in einem Bereich von Raumtemperatur bis 50° C. Das erhaltene offenkettige Amin oder Hydroxylamin (vergleiche Formel B und C) cyclisiert spontan zum gewünschten Endprodukt.

Verbindungen der Formeln I oder II, worin n die Zahl 1 bedeutet, können aus Verbindungen der Formel I oder II, worin n die Zahl 0 bedeutet, erhalten werden, und zwar durch Behandeln mit einer Persäure in an sich bekannter Weise.

Der Ausdruck »pharmazeutisch annehmbare Salze« umfaßt sowohl Salze mit anorganischen als auch Salze mit orgnaischen pharmazeutisch annehmbaren starken Säuren, wie Schwefelsäure, Salzsäure, Salpetersäure, Methansulfonsäure und p-Toluolsulfonsäure. Solche Salze können im Hinblick auf den Stand der Technik und auf die Natur der Verbindung, von welcher ein Salz gewünscht wird, von jedem Fachmann leicht hergestellt werden.

Bevorzugte Verbindungen der Formeln I und II sind solche, worin $R_1$, $R_5$ und $R_6$ obige Bedeutung besitzen, und $R_2$ und $R_3$ Wasserstoff oder niederes Alkyl bedeuten.

Ganz besonders bevorzugte Verbindungen der Formeln I und II sind:

8-Chlor-6-(2-fluorphenyl)-2H,4H-pyrazolo[3,4-d][2]benzazepin,
8-Chlor-2-(2-hydroxyäthyl)-6-phenyl-2H,4H-pyrazolo[3,4-d][2]benzazepin,
6-(2-Chlorphenyl)-3-methyl-8-nitro-2H,4H-pyrazolo[3,4-d][2]benzazepin und
8-Chlor-N-methyl-6-phenyl-2H,4H-pyrazolo[3,4-d][2]benzazepin-2-carboxamid.

Die pharmazeutischen Eigenschaften der erfindungsgemäßen Verbindungen werden durch die nachfolgenden pharmakologischen Daten erläutert.

Tabelle

| Verbindungen | Kampftest | Test an der geneigten Ebene | Test an der unanästhesierten Katze |
|---|---|---|---|
| 8-Chlor-2-/(methylamino)carbonyl/-6-phenyl-4H-pyrazolo[3,4-d][2]-benzazepin | 25 mg/kg | 400 mg/kg | 20 mg/kg |
| 6-(2-Chlorphenyl)-3-methyl-8-nitro-2H,4H-pyrazolo[3,4-d][2]benzazepin Toxizität ($DL_{50}$) = 1000 mg/kg (PO) | | 400 mg/kg | 250 mg/kg |
| 8-Chlor-6-(2-chlorphenyl)-2H,4H-pyrazolo[3,4-d][2]benzazepin Toxizität ($DL_{50}$) = 800 mg/kg (PO) | 25 mg/kg | 143 mg/kg | 10 mg/kg |
| 8-Chlor-2-(2-hydroxyäthyl)-6-phenyl-2H,4H-pyrazolo[3,4-d][2]benzazepin Toxizität ($DL_{50}$) = 750 mg/kg (PO) | 100 mg/kg | 40 mg/kg | 20 mg/kg |
| 8-Chlor-6-(2-fluorphenyl)-2H,4H-pyrazolo[3,4-d][2]benzazepin Toxizität ($DL_{50}$) = 760 mg/kg (PO) | 25 mg/kg | 66 mg/kg | 2 mg/kg |

Nachfolgend werden die an sich bekannten pharmakologischen Versuche kurz zusammengefaßt:

## Kampftest

Man bringt zwei Mäuse unter ein umgekehrtes 1-Liter-Becherglas auf ein Gitter, durch welches ihnen Schläge auf die Füße versetzt werden. Dadurch geraten die Tiere in einen Erregungszustand, welcher sich in gelegentlichen kurzen Zweikämpfen äußert. Man wählt Mäusepaare, welche während einer 2minütigen Versuchsperiode mindestens fünfmal gekämpft haben, verabreicht ihnen die Versuchssubstanz auf oralem Wege und wiederholt den Versuch nach 1 Stunde. Hierbei verwendet man logarithmisch ansteigende Dosen bis zu maximal 100 mg/kg. Man definiert als 100%ige hemmende Dosis diejenige Dosis, welche bei 3 von 3 Mäusepaaren einen Kampf verhindert.

## Test an der geneigten Ebene

Man verabreicht jeweils 6 Mäusen die Prüfsubstanz (maximale Dosis ist 500 mg/kg) und beobachtet sodann während 4 Stunden das Verhalten der Mäuse an der geneigten Ebene auf paralytische Erscheinungen, die ein Abgleiten von der Ebene verursachen. Wird eine Wirkung beobachtet, so verwendet man weitere Dosen, bis man mindestens zwei Dosen hat, bei welchen einige, jedoch nicht alle Tiere von der geneigten Ebene abgleiten. Dosen, bei welchen die Tiere auf Grund von toxischen Erscheinungen oder Erregungszuständen abgleiten, werden für die Berechnung des $PD_{50}$-Wertes nicht berücksichtigt.

## Versuch an der wachen Katze

Man behandelt Katzen auf oralem Wege und beobachtet, ob Symptome auftreten (gewöhnlich Ataxie). Zunächst verwendet man eine Katze und eine Dosis von 50 mg/kg. Wenn sich eine Wirkung zeigt, so variiert man die Dosen und benützt bis zu 3 Kapseln pro Dosis. Die Resultate zeigen die minimale wirksame Dosis.

Die erfindungsgemäßen Pyrazolo[3,4-d][2]benzazepine der obigen Formel I und deren pharmazeutisch annehmbare Säureadditionssalze sind nützlich als Heilmittel und zeichnen sich durch sedative und anxiolytische Aktivität aus. Diese Verbindungen können in Form üblicher pharmazeutischer Zubereitungen verwendet werden; beispielsweise können sie mit üblichen organischen oder anorganischen inerten, für parenterale oder enterale Verabreichung geeigneten Trägerstoffen vermischt werden, beispielsweise mit Wasser, Gelatine, Milchzucker, Stärke, Magnesiumstearat, Talk, pflanzlichen Ölen, Gummi arabicum, Polyalkylenglykolen, Vaselin oder dergleichen. Sie können als übliche pharmazeutische Formen verabreicht werden, beispielsweise als feste Form, wie Tabletten, Dragées, Kapseln, Suppositorien oder dergleichen, oder als flüssige Formen, wie Lösungen, Suspensionen oder Emulsionen. Darüber hinaus können die erfindungsgemäße Verbindungen enthaltenden pharmazeutischen Zubereitungen üblichen pharmazeutischen Operationen, wie Sterilisierung, unterzogen werden, und sie können übliche pharmazeutische Hilfsstoffe enthalten, wie Konservierungsmittel, Stabilisierungsmittel, Netzmittel, Emulgiermittel, Salze zur Veränderung des osmotischen Druckes oder Puffer. Die pharmazeutischen Präparate können auch noch andere therapeutisch wertvolle Stoffe enthalten.

Eine geeignete pharmazeutische Dosierungseinheit kann zwischen 1 bis 500 mg einer Verbindung der Formel I oder eines pharmazeutisch annehmbaren Säureadditionssalzes davon enthalten, wobei ein Dosierungsbereich von 1 mg bis 100 mg für orale Verabreichung und ein Dosierungsbereich von 1 mg bis 50 mg für parenterale Verabreichung bevorzugt ist. In jedem Einzelfall muß jedoch die Person, welche die Verabreichung der fraglichen Verbindungen durchführt oder überwacht, die Dosierung nach ihrem fachlichen Urteil den individuellen Erfordernissen anpassen. Es ist zu beachten, daß die vorstehenden Dosierungen lediglich im Sinn von Beispielen angegeben sind und daß sie den Umfang und die Ausführung der vorliegenden Erfindung in keiner Weise einschränken.

Der Ausdruck »Dosierungseinheit«, wie er in der vorliegenden Beschreibung verwendet wird, bezeichnet einzelne pharmazeutische Einheiten, welche sich als Einheitsdosen für Säuger eignen und von welchen jede eine vorbestimmte Menge des Wirkstoffs enthält, welche so berechnet wurde, daß sie zusammen mit den erforderlichen pharmazeutischen Verdünnungsmittel, Träger oder Vehikel den erwünschten therapeutischen Effekt zur Folge hat.

Die nachfolgenden Beispiele sollen die Erfindung illustrieren, jedoch nicht einschränken. Alle Temperaturen sind, sofern nichts anderes erwähnt, in Celsiusgraden angegeben.

### Beispiel 1

Man versetzt eine Lösung von 5,0 g Kupfersulfat in 3 l konzentriertem Ammoniak mit 300 g (4,6 Mol) aktiviertem Zinkstaub und 100 g (0,42 Mol) 2-Benzoyl-4-chlorbenzoesäure und erhitzt die erhaltene Mischung während 3 Tagen unter Rückfluß zum Sieden, wobei man das Volumen durch Zugabe von konzentriertem Ammoniak konstant hält. Man kühlt ab, filtriert vom überschüssigen Zink ab, säuert das Filtrat durch Zugabe von konzentrierter Salzsäure auf pH 3 an und filtriert den erhaltenen Niederschlag ab. Nach Trocknen bis zum konstanten Gewicht erhält man 2-Benzyl-4-chlorbenzoesäure als weißen Festkörper vom Schmelzpunkt 142—144°.

### Beispiel 2

Man versetzt eine auf 0° gekühlte Lösung von 28,4 g (0,75 Mol) Lithiumaluminiumhydrid in 800 ml Äther tropfenweise mit 85,1 g (0,345 mMol) 2-Benzyl-4-chlorbenzoesäure in 250 ml Äther. Man läßt auf Raumtemperatur erwärmen, rührt während 2 Stunden, zerstört das überschüssige Lithiumaluminiumhydrid durch Zugabe von 28,5 ml Wasser, 28,5 ml 10%iger wäßriger Natronlauge und 85,5 ml Wasser und filtriert vom ausgefallenen Material ab. Man trocknet das Filtrat über Natriumsulfat, entfernt den Äther unter vermindertem Druck und erhält 2-Benzyl-4-chlorbenzylalkohol als farbloses Öl, das beim Stehen kristallisiert und dann einen Schmelzpunkt von 46,5—49° aufweist.

### Beispiel 3

Man versetzt eine Suspension von 238 g (1,1 Mol) Pyridiniumchlorchromat in 800 ml Methylenchlorid mit 79,3 g (0,34 Mol) 2-Benzyl-4-chlorbenzylalkohol, rührt die Mischung während 2 Stunden bei Raumtemperatur, fällt die Chromsalze durch Zugabe von 2,4 l einer Mischung von Äther und Petroläther (1 : 1) aus und entfernt das ausgefallene Material durch Filtrieren über Kieselgur. Nach Entfernen des Lösungsmittels unter vermindertem Druck erhält man 2-Benzyl-4-chlorbenzaldehyd als gelbes Öl, das ohne weitere Reinigung in die nächste Stufe eingesetzt wird.

### Beispiel 4

Man versetzt eine Suspension von 10,5 g (0,437 Mol) Natriumhydrid (frei von Mineralöl) in 1,2 l Tetrahydrofuran tropfenweise mit 58,4 g (0,328 Mol) Diäthylcyanomethylphosphonat, versetzt, nach Beendigung der Wasserstoffentwicklung (nach ca. 60 Minuten), tropfenweise mit 69,4 g (0,3 Mol) 2-Benzyl-4-chlorbenzaldehyd in 75 ml Tetrahydrofuran und rührt die erhaltene Mischung über Nacht bei Raumtemperatur. Die Tetrahydrofuranlösung wird abdekantiert und bei Raumtemperatur eingedampft. Man verteilt den Rückstand zwischen 2 l Wasser und 1,5 l Äther, trennt die Ätherlösung ab, wäscht sie mit Wasser und trocknet sie über Natriumsulfat. Nach Entfernen des Äthers unter vermindertem Druck erhält man 3-[2-Benzyl-4-chlorphenyl]-2-propennitril als gelbes Öl, das ohne weitere Reinigung in die nächste Stufe eingesetzt wird.

Beispiel 5

Eine Mischung aus 0,4 Mol Diazomethan in 400 ml Äther und 18,2 g (0,068 Mol) 3-(2-benzoyl-4-chlorphenyl)-2-propennitril läßt man über Nacht bei Raumtemperatur stehen. Man zerstört das überschüssige Diazomethan durch tropfenweise Zugabe von 30 ml Essigsäure, wäscht die Lösung mit 5%igen wäßrigem Natriumcarbonat und Wasser, trocknet über Natriumsulfat und dampft unter vermindertem Druck zu einem gelben Öl ein.

Man rührt eine Mischung von 21,0 g dieses gelben Öles, 11,1 g (0,069 Mol) Brom, 17 g (0,204 Mol) Natriumbicarbonat und 300 ml Chloroform über Nacht bei Raumtemperatur, zerstört das überschüssige Brom durch Zugabe von 200 ml einer gesättigten wäßrigen Natriumbisulfitlösung, trennt die Chloroformlösung ab, wäscht sie mit Wasser und trocknet sie über Natriumsulfat. Durch Eindampfen der Chloroformlösung unter vermindertem Druck erhält man 4-[2-Benzoyl-4-chlorphenyl]pyrazol-3-carbonitril als gelbes Öl, das man durch Säulenchromatographie von 800 g Kieselgel unter Eluieren mit Methylenchlorid/Äther (4 : 1) reinigt. Man erhält einen weißen Festkörper vom Schmelzpunkt 194—195°.

Beispiel 6

Man hydriert eine Mischung von 2,2 g (7,2 Mol) 4-[2-Benzoyl-4-chlorphenyl]pyrazol-3-carbonitril, 2,0 g Raney-Nickel und 150 ml Essigsäure in einem Parr-Apparat während 4 Stunden. Man filtriert vom Raney-Nickel ab und verdünnt das Filtrat mit 600 ml Eiswasser. Die Essigsäure wird mit konzentriertem Ammoniak neutralisiert, und die erhaltene wäßrige Lösung wird mit Methylenchlorid ausgeschüttelt. Man wäscht die Methylenchloridlösung mit Wasser, trocknet über Natriumsulfat und dampft ein. Der erhaltene braune Festkörper wird aus Chloroform/Hexan umkristallisiert, wobei man 8-Chlor-6-phenyl2H,4H-pyrazolo[3,4-d][2]-benzazepin als weißen Festkörper vom Schmelzpunkt 241—243° erhält.

Beispiel 7

Man gibt eine Lösung von Diazoäthan in 400 ml Äther (hergestellt aus 16,1 g (0,1 Mol) N-Äthyl-N'-nitro-N-nitrosoiminoharnstoff) zu einer Lösung von 15 g (0,056 Mol) rohem 3-(2-Benzoyl-4-chlorphenyl)-2-propennitril in 100 ml Äther, läßt während 3 Stunden bei Raumtemperatur stehen und zerstört das überschüssige Diazoäthan durch Zugabe von Eisessig. Man wäscht die Mischung mit gesättigtger Natriumbicarbonatlösung, trocknet und dampft ein, wobei man den erhaltenen Rückstand in Toluol aufnimmt und erneut eindampft. Man nimmt den Rückstand in 400 ml Toluol auf und erhitzt die erhaltene Lösung während 1,5 Stunden unter Rückfluß zum Sieden, und zwar in Gegenwart von 60 g aktiviertem Mangandioxid. Man entfernt das gebildete Reaktionswasser in einem Dean-Stark-Apparat. Man filtriert anschließend durch Kieselgur, um das Mangandioxid zu entfernen, und dampft ein. Der Rückstand wird an 300 g Kieselgel unter Eluieren mit 10% Essigester in Methylenchlorid (v/v) chromatographiert. Die homogenen Fraktionen werden vereinigt und eingedampft, wobei man 4-(2-Benzoyl-5-chlorphenyl)-3-cyano-5-methylpyrazol als Öl erhält.

Beispiel 8

Man hydriert eine Mischung von 1,8 g harzartigem 4-(2-Benzoyl-4-chlorphenyl)-3-cyano-5-methylpyrazol, 35 ml Eisessig und ca. 10 g Raney-Nickel während 6 Stunden bei Raumtemperatur. Nach Abfiltrieren des Katalysators wird das Filtrat eingedampft, und der Rückstand zwischen Methylenchlorid/Äther und 10%iger wäßriger Natriumcarbonatlösung verteilt. Die organische Phase wird getrocknet und eingedampft. Man kristallisiert den Rückstand aus Äther und erhält rohes 8-Chlor-1-methyl-6-phenyl-2H,4H-pyrazolo[3,4-d][2]benzazepin, das man aus Äther umkristallisiert. Man erhält weißliche Kristalle vom Schmelzpunkt 145—150° (Schaumbildung).

Die Mutterlauge wird an 30 g Kieselgel unter Eluieren mit Essigester/Methylenchlorid (3 : 1) chromatographiert. Durch Kristallisieren des erhaltenen Stoffes aus Äther/Hexan erhält man zusätzliches Material. Dieses Material wird mit dem in Absatz 1 erhaltenen vereinigt und in heißem Äthanol aufgelöst. Man versetzt mit äthanolischem Chlorwasserstoff und kristallisiert durch Zugabe von Äther. Die ausgefallenen gelben Nadeln werden abfiltriert und getrocknet. Man erhält 8-Chlor-1-methyl-6-phenyl-2H,4H-pyrazolo[3,4-d][2]benzazepin-hydrochlorid vom Schmelzpunkt 290—295°. Durch Umkristallisieren aus Methanol/Äther erhält man eine Probe von analytischer Reinheit.

Beispiel 9

Man gibt 2 ml Methylisocyanat zu einer Lösung von 0,9 g (3,07 mMol) 8-Chlor-6-phenyl-2H,4H-pyrazolo[3,4-d][2]benzazepin in 50 ml Methylenchlorid und läßt die Mischung während 2 Stunden bei Raumtemperatur stehen. Man entfernt das Lösungsmittel und kristallisiert den Rückstand aus Essigester, wobei man 8-Chlor-N-methyl-6-phenyl-2H,4H-pyrazolo[3,4-d][2]benzazepin-2-carboxamid als weiße Nadeln vom Schmelzpunkt 230—235° erhält.

Die Mutterlauge wird eingedampft, und der Rückstand wird an 65 g Kieselgel unter Eluieren mit 20% Äther in Benzol (v/v) chromatographiert. Durch Eindampfen der einheitlichen, vereinigten Fraktionen und Kristallisieren des Rückstandes aus Essigester/Hexan erhält man 8-Chlor-N-methyl-6-phenyl3H,4H-pyrazolo[3,4-d][2]benzazepin-3-carboxamid mit den Schmelzpunkten 188—190° und 248—250° (Zersetzung).

Beispiel 10

Man versetzt eine auf —20° gekühlte Lösung von 1,2 g (4,1 mMol) 8-Chlor-6-phenyl-2H,4H-pyrazolo[3,4-d][2]benzazepin in 35 ml trockenem Tetrahydrofuran mit 9,0 ml (4,5 mMol) einer 0,5 N-Lösung von Lithiumdiisopropylamid in Tetrahydrofuran. Man läßt die Mischung auf Raumtemperatur erwärmen, versetzt anschließend mit 0,5 ml (4,5 mMol) Äthylbromacetat, rührt während 5 Stunden bei Raumtemperatur, gießt auf 100 ml Wasser und extrahiert mit Äther. Man trocknet die Ätherlösung über Natriumsulfat und dampft unter vermindertem Druck zu einem gelben Schaum ein. Den Rückstand reinigt man durch Säulenchromatographie an 30 g Kieselgel unter Eluieren mit Methylenchlorid/Äther (9 : 1) und erhält 2 Substanzen: 8-Chlor-6-phenyl-3H,4H-pyrazolo[3,4-d][2]benzazepin-3-essigsäureäthylester als gelben Schaum und in den späteren Fraktionen 8-Chlor-6-phenyl-2H,4H-pyrazolo[3,4-d][2]benzazepin-2-essigsäureäthylester als gelben Schaum.

Beispiel 11

Durch Erhitzen einer Mischung von 0,8 g (2,1 mMol) 8-Chlor-6-phenyl-2H,4H-pyrazolo[3,4-d][2]benzazepin-2-essigsäureäthylester und 20 ml methanolischem Ammoniak (ca. 20%, v/v) in einem Autoklaven auf dem Dampfbad während 20 Stunden und kristallisieren aus Essigester/Äther erhält man 8-Chlor-6-phenyl-2H,4H-pyrazolo[3,4-d][2]benzazepin-2-acetamid als farblose Kristalle vom Schmelzpunkt 236—238°. Durch Umkristallisieren aus Essigester/Methylenchlorid/Äther erhält man eine analytisch reine Probe

Beispiel 12

Man erhitzt eine Mischung von 0,4 g (1,05 mMol) 8-Chlor-6-phenyl-3H,4H-pyrazolo[3,4-d][2]benzazepin-3-essigsäureäthylester und 20 ml methanolischem Ammoniak (ca. 20%, v/v) in einem Autoklaven auf einem Dampfbad über Nacht. Man entfernt das Lösungsmittel und reinigt den erhaltenen Rückstand durch Chromatographieren an 15 g Kieselgel unter Eluieren mit 5% Äthanol in Methylenchlorid (v,v). Durch Kristallisieren aus Essigester/Äther erhält man 8-Chlor-6-phenyl-3H,4H-pyrazolo[3,4-d][2]benzazepin-3-acetamid als farblose Kristalle vom Schmelzpunkt 218—219°.

Beispiel 13

Man versetzt eine auf —30° gekühlte Lösung von 0,4g (0,01 Mol) Lithiumaluminiumhydrid in 80 ml Äther mit einer Lösung von 0,8 g (2,1 mMol) 8-Chlor-6-phenyl-2H,4H-pyrazolo[3,4-d][2]benzazepin-2-essigsäureäthylester in 5 ml Tetrahydrofuran und rührt die erhaltene Mischung während 15 Minuten bei —15° bis —5°. Man hydrolysiert durch Zugabe von 2 ml Wasser, verdünnt mit Methylenchlorid und filtriert durch Kieselgur. Nach Eindampfen des Filtrates wird der Rückstand aus Äther kristallisiert und liefert 8-Chlor-2-(2-hydroxyäthyl)-6-phenyl-2H,4H-pyrazolo[3,4-d][2]benzazepin als farblose Kristalle, welche für die Analyse aus Essigester/Hexan umkristallisiert werden. Man erhält Material vom Schmelzpunkt 173—175°.

Beispiel 14

Man rührt eine Mischung von 28,8 g (0,14 Mol) 3-[2-Benzyl-4-chlorphenyl]-2-propennitril, 50 g (0,5 Mol) Chromtrioxid, 100 ml Methylenchlorid und 300 ml Essigsäure über Nacht bei Raumtemperatur, zerstört das überschüssige Chromtrioxid durch langsame Zugabe von 30 ml Äthanol, verdünnt mit 800 ml Wasser und extrahiert mit 500 ml Äther. Man wäscht die Ätherlösung mit Wasser, gesättigter wäßriger Natriumbicarbonatlösung und gesättigter Kochsalzlösung. Die Ätherlösung wird über wasserfreiem Natriumsulfat getrocknet und unter vermindertem Druck eingedampft. Man erhält 3-(2-Benzoyl-4-chlorphenyl)-2-propennitril als gelbes Öl, das man ohne weitere Reinigung in die nächste Stufe einsetzt.

Eine Probe dieses Materials wird durch präparative Schichtchromatographie an einer 2 mm dicken Kieselgelschicht und unter Entwickeln mit Methylenchlorid/Pentan (1 : 1) gereinigt. Man erhält einen weißen Festkörper vom Schmelzpunkt 87—89°.

Beispiel 15

Man gibt eine Lösung von 92,7 g (0,4 Mol) 2-Amino-5-chlorbenzophenon in 250 ml Acetonitril zu einer Mischung von 70 g (0,52 Mol) Kupferchlorid, 65 g (0,63 Mol) t-Butylnitrit, 500 ml Acrylnitril und 500 ml

Acetonitril. Nach beendeter Zugabe rührt man noch während 2 Stunden bei Raumtemperatur, verdünnt die erhaltene Mischung mit 80 ml 6 N-Salzsäure und 1,5 l Wasser, extrahiert mit Äther und trocknet die Ätherlösung über wasserfreiem Natriumsulfat. Die Ätherlösung wird unter vermindertem Druck zu einem braunen Öl eingedampft, das α,4-Dichlor-2-(benzoyl)benzolpropannitril und 2,5-Dichlorbenzophenon enthält. Durch Verreiben des Öls mit einer Mischung von Äther und Petroläther erhält man α,4-Dichlor-2-(benzoyl)benzolpropannitril als braunen Festkörper. Durch Umkristallisieren einer kleinen Portion dieses Stoffes aus einer Mischung von Äther und Petroläther erhält man hellgelbe Nadeln vom Schmelzpunkt 69—71°.

Beispiel 16

α,4-Dichlor-2-(2-fluorbenzoyl)benzolpropannitril wird auf die gleiche Weise hergestellt wie α,4-Dichlor-2-(benzoyl)benzolpropannitril. Man erhält hellgelbe Prismen vom Schmelzpunkt 94—95°.

Beispiel 17

α,4-Dichlor-2-(2-chlorbenzoyl)benzolpropannitril wird auf die gleiche Weise hergestellt wie α,4-Dichlor-2-(benzoyl)benzolpropannitril. Man erhält weißliche Prismen vom Schmelzpunkt 102—103°.

Beispiel 18

Man rührt eine Mischung von 50,9 g (0,168 Mol) α,4-Dichlor-2-(benzoyl)benzolpropannitril, 17 g (0,14 Mol) Kaliumcarbonat, 50,9 g (0,5 Mol) Kaliumbicarbonat und 510 ml Dimethylsulfoxid während 48 Stunden bei Raumtemperatur, verdünnt die erhaltene Mischung mit 1,5 l Wasser und filtriert das ausgefallene Material ab. Durch Umkristallisieren aus einer Mischung von Methylenchlorid und Äther erhält man 3-(2-Benzoyl-4-chlorphenyl)-2-propennitril als weißliche Prismen vom Schmelzpunkt 89—91°.

Beispiel 19

3-[2-(2-Fluorbenzoyl)-4-chlorphenyl]-2-propennitril wird auf die gleiche Weise hergestellt wie 3-(2-Benzoyl-4-chlorphenyl)-2-propennitril. Man erhält weißliche Prismen von Schmelzpunkt 137—139°.

Beispiel 20

3-[2-(2-Chlorbenzoyl)-4-chlorphenyl]-2-propennitril wird auf die gleiche Weise hergestellt wie 3-(2-Benzoyl-4-chlorphenyl)-2-propennitril. Man erhält weißliche Prismen vom Schmelzpunkt 140—141°.

Beispiel 21

Eine Mischung von 7,0 g (24,5 mMol) 3-[4-Chlor-2-(2-fluorbenzoyl)phenyl)-2-propennitril, 100 ml (100 mMol) einer 1 M-Lösung von Diazomethan in Äther und 75 ml Methylenchlorid wird während 6 Stunden bei Raumtemperatur stehengelassen. Man zerstört das überschüssige Diazomethan durch Zugabe von Essigsäure, wäscht die erhaltene Lösung mit 5%iger wäßriger Natriumbicarbonatlösung, trocknet über wasserfreiem Natriumsulfat und dampft unter vermindertem Druck zu einem bernsteinfarbenen Öl ein.

Eine Mischung von 8,5 g dieses bernsteinfarbenen Öls, 2,0 ml (36,5 mMol) Brom und 150 ml Chloroform wird während 45 Minuten bei Raumtemperatur gerührt. Man entfernt die flüchtigen Anteile unter vermindertem Druck und nimmt den Rückstand in 100 ml Chloroform auf. Man erhitzt die Chloroformlösung während 30 Minuten auf dem Dampfbad zum Sieden, wäscht anschließend mit 5%iger wäßriger Natriumbicarbonatlösung, trocknet über wasserfreiem Natriumsulfat und dampft unter vermindertem Druck zu einem bernsteinfarbenen Öl ein. Man reinigt den Rückstand durch Säulenchromatographie an 100 g Kieselgel unter Eluieren mit 5% Äther in Methylenchlorid und erhält als Hauptprodukt 4-[4-Chlor-2-(2-fluorbenzoyl)phenyl]-1H-pyrazol-3-carbonitril als hellgelbe Prismen vom Schmelzpunkt 169—170°.

Beispiel 22

4-[2-(2-Chlorbenzoyl)-4-chlorphenyl]-1H-pyrazol-3-carbonitril wird auf die gleiche Weise hergestellt wie 4-[4-Chlor-2-(2-fluorbenzoyl)phenyl]-1H-pyrazol-3-carbonitril. Man erhält hellgelbe Prismen vom Schmelzpunkt 183—183°.

Beispiel 23

Eine Mischung von 2,0 g (6,1 mMol) 4-[4-Chlor-2-(2-fluorbenzoyl)phenyl]-1H-pyrazol-3-carbonitril, etwa 2 g Raney-Nickel und 100 ml Essigsäure wird während 4 Stunden in einem Parr-Apparat hydriert. Das Raney-Nickel wird abfiltriert und das Filtrat wird unter vermindertem Druck zu einem gelben Öl eingedampft. Dieses gießt man auf Eis, stellt mit Ammoniak basisch und extrahiert mit Methylenchlorid. Die Methylenchloridlösung wird mit Wasser gewaschen, über wasserfreiem Natriumsulfat getrocknet und unter vermindertem Druck zu einem hellgelben Festkörper eingedampft. Durch Umkristallisieren aus einer Mischung aus Äther und Methylenchlorid erhält man 8-Chlor-6-(2-fluorphenyl)-2H,4H-pyrazolo[3,4-d][2]benzazepin als crèmefarbene Prismen vom Schmelzpunkt 208—210°.

Beispiel 24

8-Chlor-6-(2-chlorphenyl)-2H,4H-pyrazolo[3,4-d][2]benzazepin wird auf die gleiche Weise hergestellt wie 8-Chlor-6-(2-fluorphenyl)-2H,4H-pyrazolo[3,4-d][2]benzazepin. Man erhält mit Methylenchlorid solvatisiertes Endprodukt als crèmefarbene Prismen vom Schmelzpunkt 142—144° (Schaumbildung).

Beispiel 25

Eine Lösung von 5,0 g (14 mMol) 5-Chlor-2'-fluor-2-jodbenzophenon, 2 ml (14,3 mMol) Triäthylamin, 2 ml (30 mMol) Acrylnitril und 35 ml (1,5 mMol) Palladiumacetat wird während 16 Stunden unter einer Argonatmosphäre unter Rückfluß zum Sieden erhitzt. Man verdünnt die erhaltene Mischung mit 100 ml 1 N-Salzsäure und filtriert den erhaltenen Niederschlag ab. Nach Waschen des Niederschlages mit Äther und Trocknen an der Luft erhält man 3-[4-Chlor-2-(2-fluorbenzoyl)phenyl]-2-propennitril als weißlichen Festkörper vom Schmelzpunkt 130—133°.

Beispiel 26

Man versetzt 180 ml in einem Eisbad auf 0° gekühlte konzentrierte Schwefelsäure portionenweise mit 28 g (0,4 Mol) Natriumnitrit, und zwar so, daß die Reaktionstemperatur 10° nicht übersteigt. Die erhaltene Mischung wird anschließend auf dem Dampfbad erwärmt, bis man eine Lösung erhält. Anschließend kühlt man auf 30° ab und versetzt mit einer Lösung von 110,68 g (0,4 Mol) 2-Amino-5-nitro-2'-chlorbenzophenon in 300 ml heißer Essigsäure, so daß die Reaktionstemperatur 40° nicht übersteigt. Die erhaltene Mischung wird ohne weiteres Erwärmen während 2,5 Stunden gerührt und anschließend langsam auf 800 ml konzentrierte Salzsäure und 80 g Kupfer(I)chlorid gegossen. Man erhitzt während 40 Minuten auf dem Dampfbad auf 80° und gießt, nach beendeter Schaumbildung, auf 2 l Wasser und läßt über Nacht stehen. Man filtriert das ausgefallene Material ab, wäscht es mit Wasser und kristallisiert es aus Äthanol um, wobei man (2-Chlor-5-nitrophenyl)(2-chlorphenyl)methanon vom Schmelzpunkt 76—79° erhält.

Eine Probe dieses Materials wird aus Methanol umkristallisiert und hat dann einen Schmelzpunkt von 78,5—80°.

Beispiel 27

Eine Mischung von 900 ml Dimethylformamid, 165 ml Toluol, 65,6 g (1,13 Mol) wasserfreiem Kaliumfluorid und 74,6 g (0,252 Mol) (2-Chlor-5-nitrophenyl)(2-chlorphenyl)methanon wird unter Rühren und Rückfluß zum Sieden erhitzt und durch Abdestillieren von 170 ml Lösungsmittel getrocknet. Anschließend erhitzt man noch über Nacht unter Rückfluß zum Sieden, kühlt in einem Eisbad ab, verdünnt mit 1 l Wasser und 100 ml Hexan und filtriert 1 Stunde später den goldfarbenen Niederschlag ab. Man nimmt diesen in 300 ml Methylenchlorid auf, trennt das Wasser ab, trocknet die Lösung über Natriumsulfat und dampft im Vakuum ein. Der Rückstand wird in kaltem Äther aufgeschlämmt, um von unlöslichem rohem 2-Nitroxanthen-9-on abzutrennen. Nach Eindampfen der Ätherlösung im Vakuum und Umkristallisieren des Rückstandes aus Methanol erhält man (2-Chlorphenyl)(2-fluor-5-nitrophenyl)methanon vom Schmelzpunkt 60—64°. Durch Umkristallisieren einer Probe aus Methanol erhält man analytisch reines Material vom Schmelzpunkt 60—64°.

Beispiel 28

Man versetzt eine in einem Wasserbad gekühlte Mischung von 100 ml Diemethylsulfoxid und 8 ml (78 mMol) Acetylaceton mit 8,8 g Kalium-t-butoxid. Die einsetzende exotherme Reaktion wird durch Zugabe von Eis ins Kühlwasser kontrolliert. Nach 0,5 Stunden versetzt man mit 11,2 g (40 mMol) (2-Chlorphenyl)(2-fluor-5-nitrophenyl)methanon und rührt die Reaktionsmischung während 1 Stunde bei Raumtemperatur. Anschließend gießt man auf eine gerührte Mischung von 200 ml Eiswasser, 40 ml 3 N-Salzsäure und wenig Äther. Eine Stunde später wird das ausgefallene Material abfiltriert, mit

Wasser und Hexan gewaschen und aus Methanol umkristallisiert. Eine Probe des erhaltenen 3-(2-Chlorbenzoyl-4-nitrophenyl)-2,4-pentandions wird erneut aus Methanol umkristallisiert und hat dann einen Schmelzpunkt von 138—140°.

Beispiel 29

Eine Lösung von 9 g (25 mMol) 3-(2-Chlorbenzoyl-4-nitrophenyl)-2,4-pentandion in 60 ml einer 0,5 M-Lösung von Hydrazin-hydrochlorid in wäßrigem Äthanol wird über Nacht unter Rückfluß zum Sieden erhitzt und anschließend im Vakuum eingedampft. Man verteilt den Rückstand zwischen 100 ml Methylenchlorid und 50 ml Wasser, stellt den pH der wäßrigen Phase mit konzentriertem Amoniak auf 5 ein, trennt die organische Phase ab und wäscht sie mit 50 ml Wasser. Nach Trocknen über Natriumsulfat und Eindampfen im Vakuum wird der Rückstand aus Äther/Hexan kristallisiert und aus Essigester/Hexan umkristallisiert. Eine Probe des so erhaltenen [5-Nitro-2-(3,5-dimethyl-1H-pyrazol-4-yl)phenyl]-2-chlorphenyl-methanons wird aus Essigester umkristallisiert und hat dann einen Schmelzpunkt von 160—162°.

Beispiel 30

Eine Mischung von 3,2 ml Methylhydrazin und 80 ml Äthanol wird mit 7,4 ml konzentrierter Salzsäure und 14,4 g (40 mMol) 3-(2-Chlorbenzoyl-4-nitrophenyl)-2,4-pentandion versetzt. Man rührt und erhitzt die Mischung während 6 Stunden unter Rückfluß zum Sieden, dampft anschließend im Vakuum ein, verteilt den Rückstand zwischen Methylenchlorid und Wasser und stellt den pH der wäßrigen Phase mit Natriumbicarbonat auf 6 ein. Die organische Phase wird abgetrennt, über Natriumsuflat getrocknet und im Vakuum eingedampft. Den Rückstand chromatographiert man an Kieselgel unter Eluieren mit Methylenchlorid und Methylenchlorid, das 2% Methanol enthält. Die Fraktionen, welche das gewünschte (2-Chlorphenyl)[5-nitro-2-(1,3,5-trimethyl-1H-pyrazolyl)]methanon enthalten, werden vereinigt. Das daraus gewonnene Material kristallisiert man aus Essigester und erhält Material vom Schmelzpunkt 146—150°. Eine Probe dieses Materials wird aus Essigester/Hexan umkristallisiert und liefert (2-Chlorphenyl)[5-nitro-2-(1,3,5-trimethyl-1H-pyrazolyl)]methanon vom Schmelzpunkt 146—149°.

Beispiel 31

Man versetzt eine Lösung von 6,2 g (57,1 mMol) t-Butylhypochlorit in 250 ml Methylenchlorid mit 18,39 g (51,7 mMol) des Endproduktes von Beispiel 29, läßt die Reaktionsmischung während 6 Stunden bei Raumtemperatur stehen und dampft anschließend im Vakuum ein. Nach Kristallisieren des Rückstandes aus Äther erhält man rohes [5-Nitro-2-[3-(chlormethyl)-5-methyl-1H-pyrazol-4-yl]phenyl]-2-chlorphenylmethanon. Eine Probe dieses Materials wird aus wäßrigem Methanol umkristallisiert und hat dann einen Schmelzpunkt von 147—149°.

Beispiel 32

Man versetzt eine Lösung von 3 g (27,6 mMol) t-Butylhypochlorit in 100 ml Methylenchlorid mit 9,25 g (25 mMol) des Endproduktes von Beispiel 30. Die Reaktionsmischung erwärmt sich dabei und beginnt zu Sieden. Man rührt über Nacht und dampft anschließend im Vakuum ein. Nach Behandeln des Rückstandes mit Äther erhält man rohes (2-Chlorphenyl)[5-nitro-2-[3-(chlormethyl)-1,5-dimethyl1H-pyrazol-4-yl]phenyl]methanon vom Schmelzpunkt 142—145°. Eine Probe dieses Materials wird aus Essigester umkristallisiert und hat dann einen Schmelzpunkt von 145—147°.

Beispiel 33

Man versetzt ca. 50 ml flüssiges Ammoniak mit 3 g (7,7 mMol) des Endproduktes von Beispiel 31, läßt das Ammoniak während 1 Stunde abdampfen und versetzt anschließend mit 25 ml Tetrahydrofuran. Diese Mischung wird während 0,5 Stunden unter Rückfluß zum Sieden erhitzt, um überschüssiges Ammoniak zu entfernen, und anschließend im Vakuum eingedampft. Durch Kristallisieren des Rückstandes aus einer Mischung aus Wasser und Äther erhält man rohes 6-(2-Chlorphenyl)-3-methyl-8-nitro-2H,4H-pyrazolo[3,4-d][2]benzazepin. Eine Probe dieses Materials wird aus Acetonitril umkristallisiert und liefert gelbe Prismen vom Schmelzpunkt 237—241° (Zersetzung).

Beispiel 34

Eine Mischung von 25 ml Tetrahydrofuran, 25 ml flüssigem Ammoniak und 1 g Natriumjodid versetzt man mit 5 g (12,4 mMol) des Endproduktes von Beispiel 32. Man rührt die Reaktionsmischung über Nacht, wobei das Ammoniak abdampft. Man verdünnt mit 100 ml Methylenchlorid, wäscht mit 50 ml Wasser, trocknet über Natriumsulfat und dampft im Vakuum zu einem gelben Teer ein. Man löst diesen

Teer und 1 ml Essigsäure in 100 ml Toluol, erhitzt während 3 Stunden unter Rückfluß zum Sieden, wobei man das Wasser in einem Dean-Stark-Apparat abtrennt, und dampft anschließend im Vakuum zu einem gelben Öl ein. Dieses wird an Kieselgel unter Eluieren mit Methanol/Methylenchlorid (0,5% bis 5%) chromatographiert. Man vereinigt die Fraktionen, welche das gewünschte Endprodukt enthalten, und behandelt sie mit 1,35 g Maleinsäure in Äther, um das entsprechende Maleat zu bilden. Das ausgefallene 6-(2-Chlorphenyl)-1,2-dimethyl-8-nitro-2H,4H-pyrazolo[3,4-d][2]benzazepin-maleat wird abfiltriert, mit Äther gewaschen und hat dann einen Schmelzpunkt von 138—142°. Eine Probe dieses Materials wird aus 2-Propanol umkristallisiert und hat dann einen Schmelzpunkt von 135—137°.

Aus einem ähnlichen Ansatz ausgehend von 17,1 g (42,3 mMol) des Endproduktes von Beispiel 32 erhält man nach Chromatographie ein Öl, das nach Kristallisieren aus Äther einen Schmelzpunkt von 140—145° aufweist. Eine Probe dieses Materials hat nach Umkristallieren aus Essigester/Hexan einen Schmelzpunkt von 141—143°.

Beispiel 35

Herstellung von Tabletten (Naßgranulierung)

| Bestandteile | mg/Tablette | mg/Tablette | mg/Tablette | mg/Tablette |
|---|---|---|---|---|
| 1. 8-Chlor-6-(2-fluorphenyl)-2H,-4H-pyrazolo[3,4-d][2]benzazepin oder 6-(2-Chlorphenyl)-3-methyl-8-nitro-2H,4H-pyrazolo[3,4-d]-[2]benzazepin | 1 | 5 | 10 | 25 |
| 2. Lactose | 202 | 232 | 261 | 280 |
| 3. Modifizierte Stärke | 25 | 35 | 45 | 55 |
| 4. Vorgelatinisierte Stärke | 20 | 25 | 30 | 35 |
| 5. Destilliertes Wasser q.s. | — | — | — | — |
| 6. Magnesiumstearat | 2 | 3 | 4 | 5 |
| Tablettengewicht | 250 | 300 | 350 | 400 |

Man mischt die Bestandteile 1—4 in einem geeigneten Mixer und granuliert mit genügend Wasser, um eine gute Konsistenz zu erhalten. Nach dem Mahlen wird in einem geeigneten Ofen getrocknet. Anschließend mahlt und vermischt man während 3 Minuten mit Magnesiumstearat. Schließlich wird das Gemisch maschinell zu Tabletten entsprechender Größe verpreßt.

Beispiel 36

Herstellung von Tabletten (direkte Verpressung)

| Bestandteile | mg/Tablette | mg/Tablette | mg/Tablette | mg/Tablette |
|---|---|---|---|---|
| 1. 8-Chlor-6-(2-fluorphenyl)-2H,-4H-pyrazolo[3,4-d][2]benzazepin oder 6-(2-Chlorphenyl)-3-methyl-8-nitro-2H,4H-pyrazolo[3,4-d]-[2]benzazepin | 1 | 5 | 10 | 25 |
| 2. Lactose | 221 | 217 | 212 | 181 |
| 3. Avicel | 45 | 45 | 45 | 55 |
| 4. Stärke zur direkten Verpressung | 30 | 30 | 30 | 35 |
| 5. Magnesiumstearat | 3 | 3 | 3 | 4 |
| Tablettengewicht | 300 | 300 | 300 | 300 |

Man mischt Bestandteil 1 mit einer äquivalenten Menge Lactose. Anschließend wird diese Mischung mit den Bestandteilen 3, 4 und dem Rest von Bestandteil 2 gut vermischt. Nach Zugabe von Magnesiumstearat mischt man während 3 Minuten. Die Mischung wird schließlich maschinell zu Tabletten entsprechender Größe verpreßt.

Beispiel 37

Herstellung von Kapseln

| Bestandteile | mg/Kapsel | mg/Kapsel | mg/Kapsel | mg/Kapsel |
|---|---|---|---|---|
| 1. 8-Chlor-6-(2-flurphenyl)-2H,-4H-pyrazolo[3,4-d][2]benzazepin oder 6-(2-Chlorphenyl)-3-methyl-8-nitro-2H,4H-pyrazolo[3,4-d]-[2]benzazepin | 1 | 5 | 10 | 25 |
| 2. Lactose | 203 | 293,5 | 328 | 372,5 |
| 3. Stärke | 30 | 35 | 40 | 30 |
| 4. Talk | 15 | 15 | 20 | 20 |
| 5. Aerosol OT | 1 | 1,5 | 2 | 2,5 |
| Kapselfüllgewicht | 250 | 350 | 400 | 450 |

Die Bestandteile 1, 2, 3 und 5 werden in einem geeigneten Mixer gut vermischt. Nach Zugabe von Talk wird wiederum gut vermischt und die Mischung mit einer geeigneten Maschine in Kapseln abgefüllt.

**Patentansprüche**

1. Pyrazolobenzazepine der allgemeinen Formeln

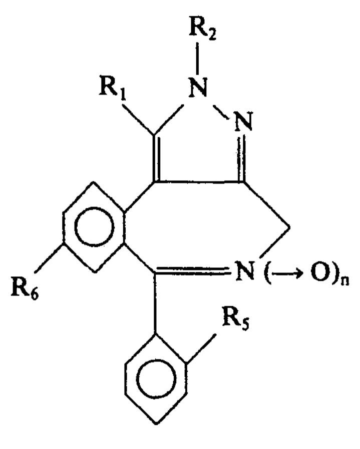

(I)

und

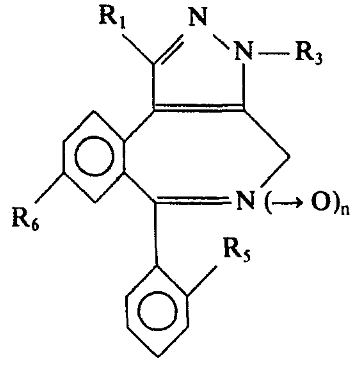

(II)

worin $R_1$ Wasserstoff oder $C_1-C_7$-Alkyl, $R_2$ und $R_3$ Wasserstoff, $C_1-C_7$-Alkyl, Hydroxy-$C_2-C_7$-Alkyl, die Gruppe $-C_1-C_6$-Alkyl-$COR_{15}$ oder die Gruppe $-COR_{11}$, $R_{11}$ $C_1-C_7$-Alkoxy, Amino oder mono-$C_1-C_7$-Alkylamino, $R_{15}$ $C_1-C_7$-Alkoxy, Amino, mono-$C_1-C_7$-Alkylamino oder di-$C_1-C_7$-Alkylamino, $R_6$ Nitro oder Halo mit einer Ordnungszahl, welche nicht größer als 35 ist, $R_5$ Wasserstoff oder Halo mit einer Ordnungszahl, welche nicht größer als 35 ist, und n die Zahl 0 oder 1 bedeuten,
und pharmazeutisch annehmbare Salze davon.

2. Verbindungen gemäß Anspruch 1, dadurch gekennzeichnet, daß $R_1$ Wasserstoff oder Methyl bedeutet, wenn $R_6$ Nitro bedeutet.

3. Verbindungen gemäß Anspruch 1 oder 2, dadurch gekennzeichnet, daß $R_2$ und $R_3$ Wasserstoff oder $C_1-C_7$-Alkyl bedeuten.

4. 8-Chlor-6-(2-fluorphenyl)-2H,4H-pyrazolo[3,4-d][2]benzazepin.

5. 8-Chlor-2-(2-hydroxyäthyl)-6-phenyl-2H,4H-pyrazolo[3,4-d][2]benzazepin.

6. 6-(2-Chlorphenyl)-3-methyl-8-nitro-2H,4H-pyrazolo[3,4-d][2]benzazepin.

7. 8-Chlor-N-methyl-6-phenyl-2H,4H-pyrazolo[3,4-d][2]benzazepin-2-carboxamid.

8. Verbindungen gemäß einem der Ansprüche 1—7 als pharmazeutische Wirkstoffe.

9. Verbindungen gemäß einem der Ansprüche 1—7 als anxiolytisch und sedativ wirksame Stoffe.

10. Verfahren zur Herstellung von Verbindungen gemäß einem der Ansprüche 1—7, dadurch gekennzeichnet, daß man

a) eine Verbindung der allgemeinen Formel

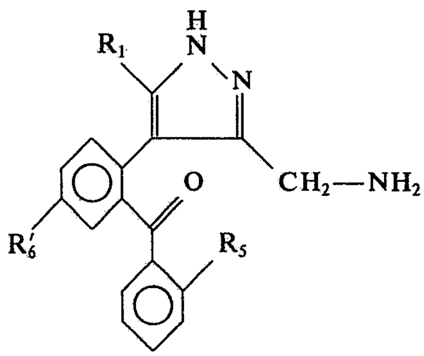

(A)

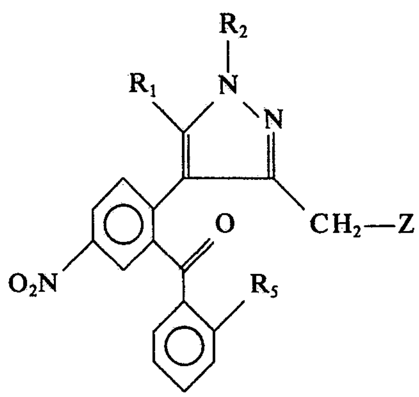

(B)

oder

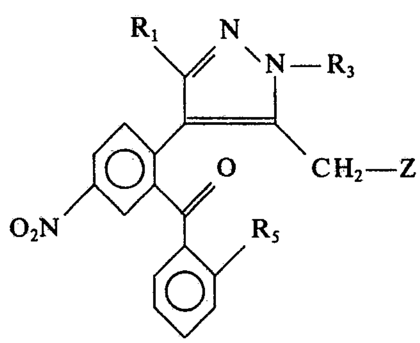

(C)

worin $R_1$, $R_2$, $R_3$ und $R_5$ die in Anspruch 1 angegebene Bedeutung besitzen, $R_6'$ Halo und Z $-NH_2$ oder $-NHOH$ bedeuten,
cyclisiert, oder

b) eine Verbindung der in Anspruch 1 definierten Formel I, worin $R_2$ Wasserstoff bedeutet, zuerst mit einer Base und anschließend mit einem einen $C_1-C_7$-Alkylrest liefernden Mittel oder einem Halo-$C_2$- bis $C_7$-Carbonsäureester behandelt, oder

c) eine Verbindung der in Anspruch 1 definierten Formel I oder II, worin $R_2$ bzw. $R_3$ $C_1$- bis $C_6$-Alkyl-COO-$C_1-C_7$-Alkyl bedeutet, mit Ammoniak oder einem mono- oder di-$C_1-C_7$-Alkylamin behandelt, oder

d) eine Verbindung der in Anspruch 1 definierten Formel I oder II, worin $R_2$ bzw. $R_3$ $C_1$- bis $C_6$-Alkyl-COO-$C_1-C_7$-Alkyl und $R_6$ Halo bedeuten, mit einem Metallhydrid-Reduktionsmittel behandelt, oder

e) eine Verbindung der in Anspruch 1 definierten Formel I, worin $R_2$ Wasserstoff und $R_6$ Halo bedeuten, mit einem $C_1-C_7$-Alkyl- oder Alkalimetallisocyanat oder zuerst mit einer Base und anschließend mit einem $C_1-C_7$-Alkyl-Haloformat behandelt, oder

f) eine Verbindung der in Anspruch 1 definierten Formel I oder II, worin n die Zahl 0 bedeutet, zu einer Verbindung der in Anspruch 1 definierten Formel I oder II, worin n die Zahl 1 bedeutet, oxydiert, oder

g) eine Verbindung der in Anspruch 1 definierten Formel I oder II in ein pharmazeutisch annehmbares Säureadditionssalz überführt.

11. Arzneimittel, enthaltend eine Verbindung gemäß einem der Ansprüche 1—7.

12. Anxiolytika und Sedativa, enthaltend eine Verbindung gemäß einem der Ansprüche 1—7.

**Claims**

1. Pyrazolobenzazepines of the general formulae

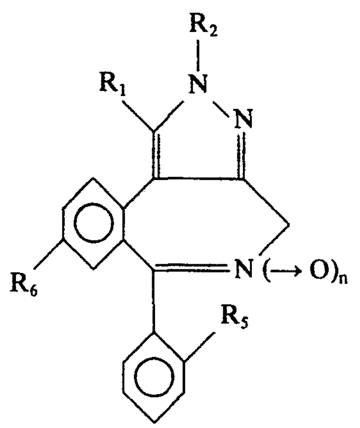

(I)

and

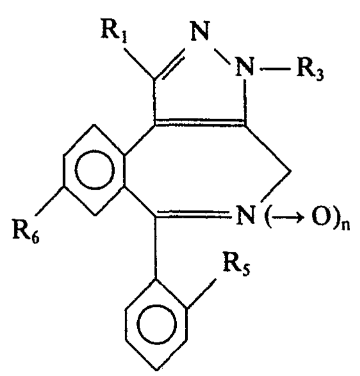

(II)

wherein $R_1$ signifies hydrogen or $C_1-C_7$-alkyl, $R_2$ and $R_3$ signify hydrogen, $C_1-C_7$-alkyl, hydroxy-$C_2-C_7$-alkyl, the group $-C_1-C_6$-alkyl-$COR_{15}$ or the group $-COR_{11}$, $R_{11}$ signifies $C_1-C_7$-alkoxy, amino or mono-$C_1-C_7$-alkylamino, $R_{15}$ signifies $C_1-C_7$-alkoxy, amino, mono-$C_1-C_7$-alkylamino or di-$C_1-C_7$-alkylamino, $R_6$ signifes nitro or halo with an atomic number which is not greater than 35, $R_5$ signifies hydrogen or halo with an atomic number which is not greater than 35, and n signifies the nunber 0 or 1,

and pharmaceutically acceptable salts thereof.

2. Compounds according to claim 1, characterized in that $R_1$ signifies hydrogen or methyl when $R_6$

signifies nitro.

3. Compounds according to claim 1 or 2, characterized in that $R_2$ and $R_3$ signify hydrogen or $C_1-C_7$-alkyl.

4. 8-Chloro-6-(2-fluorophenyl)-2H,4H-pyrazolo[3,4-d][2]benzazepine.

5. 8-Chloro-2-(2-hydroxyethyl)-6-phenyl-2H,4H-pyrazolo[3,4-d][2]benzazepine.

6. 6-(2-Chlorophenyl)-3-methyl-8-nitro-2H,4H-pyrazolo[3,4-d][2]benzazepine.

7. 8-Chloro-N-methyl-6-phenyl-2H,4H-pyrazolo[3,4-d][2)benzazepine-2-carboxamide.

8. Compounds according to any one of claims 1—7 as pharmaceutically active substances.

9. Compounds according to any one of claims 1—7 as anxiolytic and sedative active substances.

10. A process for the manufacture of compounds according to any one of claims 1—7, characterized by

a) cyclizing a compound of the general formula

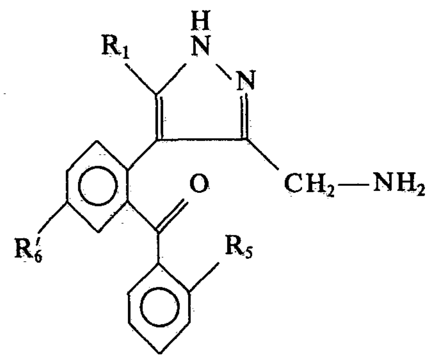

(A)

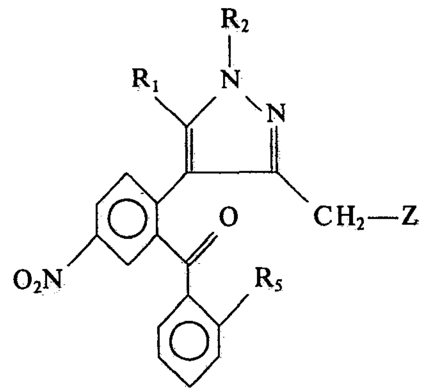

(B)

or

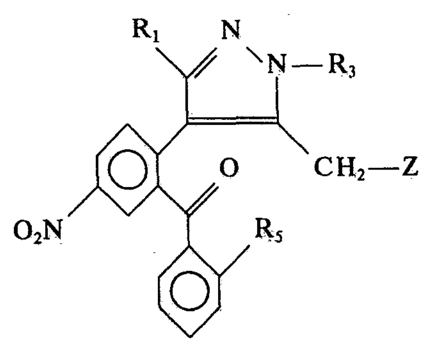

(C)

wherein $R_1$, $R_2$, $R_3$ and $R_5$ have the significance given in claim 1, $R_6'$ signifies halo and Z signifies $-NH_2$ or $-NHOH$,

or

b) treating a compound of formula I defined in claim 1 wherein $R_2$ signifies hydrogen firstly with a base and subsequently with an agent yielding a $C_1-C_7$-alkyl residue or a halo-$C_2$- to $C_7$-carboxylic acid ester, or

c) treating a compound of formula I or II defined in claim 1 wherein $R_2$ or $R_3$ signifes $C_1$- to $C_6$-alkyl-COO-$C_1-C_7$-alkyl with ammonia or a mono- or di-$C_1-C_7$-alkylamine, or

d) treating a compound of formula I or II defined in claim 1 wherein $R_2$ or $R_3$ signifes $C_1$- to $C_6$-alkyl-COO-$C_1-C_7$-alkyl and $R_6$ signifies halo with a metal hydride reduction agent, or

e) treating a compound of formula I defined in claim 1 wherein $R_2$ signifies hydrogen and $R_6$ signifies halo with a $C_1-C_7$-alkyl or alkali metal isocyanate or firstly with a base and subsequently with a $C_1-C_7$-alkyl-haloformate, or

f) oxidizing a compound of formula I or II defined in claim 1 wherein n signifies the number 0 to a compound of formula I or II defined in claim 1 wherein n signifies the number 1, or

g) converting a compound of formula I or II defined in claim 1 into a pharmaceutically acceptable acid addition salt.

11. Medicaments containing a compound according to any one of claims 1—7.

12. Anxiolytics and sedatives containing a compound according to any one of claims 1—7.

**Revendications**

1. Pyrazolobenzazépines de formules générales:

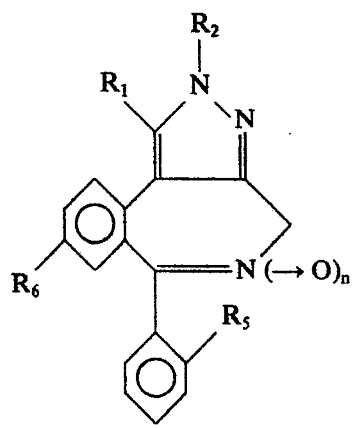

(I)

et

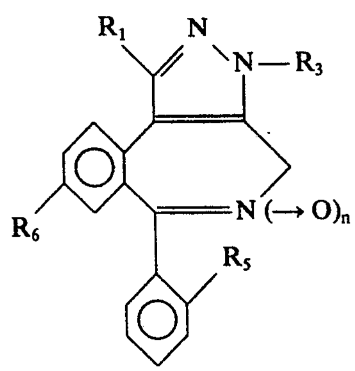

(II)

où

$R_1$ représente un hydrogène ou un alcoyle en $C_1$ à $C_7$,

$R_2$ et $R_3$ représentent un hydrogène, un alcoyle en $C_1$ à $C_7$, un hydroxyalcoyle en $C_2$ à $C_7$, le groupe alcoyle en $C_1$ à $C_6$-$COR_{15}$ ou le groupe $-COR_{11}$,

$R_{11}$ représente un alcoxy en $C_1$ à $C_7$, un amino ou un mono-alcoyle en $C_1$ à $C_7$-amino,

$R_{15}$ représente un alcoxy en $C_1$ à $C_7$, un amino, un mono-alcoyle en $C_1$ à $C_7$-amino ou un di-alcoyle en $C_1$ à $C_7$-amino,

$R_6$ représente un nitro ou un halo avec un nombre atomique qui n'est pas supérieur à 35,

$R_5$ représente un hydrogène ou un halo avec un nombre atomique qui n'est pas supérieur à 35, et

n représente le nombre 0 ou 1,

et leurs sels pharmaceutiquement acceptables.

2. Composés selon la revendication 1, caractérisés en ce que $R_1$ représente un hydrogène ou un méthyle lorsque $R_6$ représente un nitro.

3. Composés selon l'une des revendications 1 ou 2, caractérisé en ce que $R_2$ et $R_3$ représentent un hydrogène ou un alcoyle en $C_1$ à $C_7$.

4. 8-chloro-6-(2-fluorophényl)-2H,4H-pyrazolo[3,4-d][2]benzazépine.
5. 8-chloro-2-(2-hydroxyéthyl)-6-phényl-2H,4H-pyrazolo[3,4-d][2]benzazépine.
6. 6-(2-chlorophényl)-3-méthyl-8-nitro-2H,4H-pyrazolo[3,4-d][2]benzazépine.
7. 8-chloro-N-méthyl-6-phényl-2H,4H-pyrazolo[3,4-d][2]benzazépine-2-carboxamide.
8. Comkposés selon l'une des revendications 1—7 comme substances actives pharmaceutiques.
9. Composés selon l'une des revendications 1—7 comme produits anxiolytiques et à action sédative.
10. Procédé de préparation de composés selon l'une des revendications 1—7, caractérisé en ce que:

a) on cyclise un composé de formule générale:

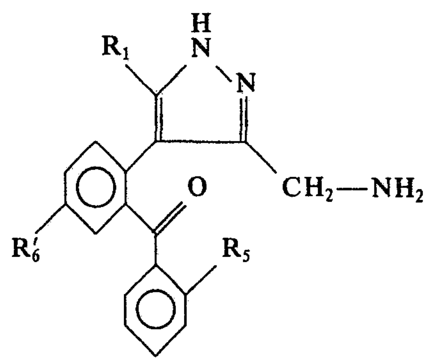

(A)

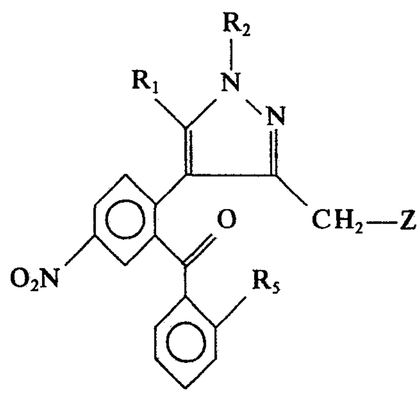

(B)

où

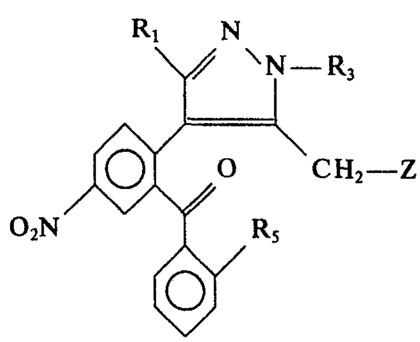

(C)

où $R_1$, $R_2$, $R_3$ et $R_5$ ont la signification donnée dans la revendication 1, $R_6'$ représente un halo et Z représente $-NH_2$ ou $-NHOH$, ou

b) on traite un composé de formule I définie dans la revendication 1, où $R_2$ représente un hydrogène, tout d'abord avec une base puis avec un agent donneur de radical alcoyle en $C_1$ à $C_7$ ou un ester d'acide halo-carboxylique en $C_2$ à $C_7$, ou

c) on traite un composé de formule I ou II définie dans la revendication 1, où $R_2$ ou selon les cas $R_3$ représente un alcoyle en $C_1$ à $C_6$—COO-alcoyle en $C_1$ à $C_7$, avec de l'ammoniac ou avec une mono- ou di-alcoyle en $C_1$ à $C_7$-amine, ou

d) on traite un composé de formule I ou II définie dans la revendication 1, où $R_2$ ou selon les cas $R_3$ représente un alcoyle en $C_1$ à $C_6$-COO-alcoyle en $C_1$ à $C_7$ et $R_6$ représente un halo, avec un agent réducteur hydrure métallique, ou

e) on trainte un composé de formule I définie dans la revendication 1, où $R_2$ représente un hydrogène et $R_6$ représente un halo, avec un isocyanate d'alcoyle en $C_1$ à $C_7$ ou de métal alcalin ou tout d'abord, avec une base puis avec un haloformiate d'alcoyle en $C_1$ à $C_7$, ou

f) on oxyde un composé de formule I ou II définie dans la revendication 1, où n représente le nombre 0, en un composé de formule I ou II définie dans la revendication 1, où n représente le nombre 1, ou

g) on transforme un composé de formule I ou II définie dans la revendication 1 en un sel d'addition d'acide pharmaceutiquement acceptable.

11. Médicaments contenant un composé selon l'une des revendications 1—7.

12. Agents anxiolytiques et sédatifs contenant un composé selon l'une des revendications 1—7.